# EUROPEAN PATENT SPECIFICATION

(11) **EP 2 126 052 B1**
(45) Date of publication and mention of the grant of the patent: **10.12.2014**
(21) Application number: 08706977.9
(22) Date of filing: 09.01.2008
(51) Int. Cl.: C12N 5/077, A61K 35/34

(54) **PROVISION OF NEW CARDIOMYOCYTE PROGENITOR CELLS AND CARDIOMYOCYTES DERIVED THEREFROM**
BEREITSTELLUNG NEUER HERZMUSKELVORLÄUFERZELLEN UND HERZMUSKELZELLEN DARAUS
OBTENTION DE NOUVELLES CELLULES PROGÉNITRICES DE CARDIOMYOCYTES ET CARDIOMYOCYTES OBTENUES À PARTIR DE CES CELLULES

(30) Priority: 09.01.2007 EP 07000374; 07.01.2008 EP 08000174
(43) Date of publication of application: 02.12.2009
(73) Proprietor: Lead Pharma Cel Models IP B.V., 6534 AT Nijmegen (NL)
(72) Inventor: GOUMANS, Marie, José, 3641 CJ Mijdrecht (NL); DOEVENDANS, Pieter, 3512 LR Utrecht (NL)
(74) Representative: Hatzmann, Martin
(86) International application number: PCT/EP2008/000119
(87) International publication number: WO 2008/083962

(56) References cited:
- OH HIDEMASA ET AL: "Cardiac progenitor cells from adult myocardium: homing, differentiation, and fusion after infarction." PROCEEDINGS OF THE NATIONAL ACADEMY OF SCIENCES OF THE UNITED STATES OF AMERICA 14 OCT 2003, vol. 100, no. 21, 14 October 2003 (2003-10-14), pages 12313-12318, XP002984517 ISSN: 0027-8424
- MATSUURA KATSUHISA ET AL: "Adult cardiac Sca-1-positive cells differentiate into beating cardiomyocytes." THE JOURNAL OF BIOLOGICAL CHEMISTRY 19 MAR 2004, vol. 279, no. 12, 19 March 2004 (2004-03-19), pages 11384-11391, XP002477754 ISSN: 0021-9258
- MESSINA ELISA ET AL: "Isolation and expansion of adult cardiac stem cells from human and murine heart." CIRCULATION RESEARCH 29 OCT 2004, vol. 95, no. 9, 29 October 2004 (2004-10-29), pages 911-921, XP002477755 ISSN: 1524-4571
- LAUGWITZ KARL-LUDWIG ET AL: "Postnatal isl1+ cardioblasts enter fully differentiated cardiomyocyte lineages." NATURE 10 FEB 2005, vol. 433, no. 7026, 10 February 2005 (2005-02-10), pages 647-653, XP002477756 ISSN: 1476-4687
- BELTRAMI ANTONIO P ET AL: "Adult cardiac stem cells are multipotent and support myocardial regeneration." CELL 19 SEP 2003, vol. 114, no. 6, 19 September 2003 (2003-09-19), pages 763-776, XP002304004 ISSN: 0092-8674
- PFISTER OTMAR ET AL: "CD31- but Not CD31+ cardiac side population cells exhibit functional cardiomyogenic differentiation." CIRCULATION RESEARCH 8 JUL 2005, vol. 97, no. 1, 8 July 2005 (2005-07-08), pages 52-61, XP002477757 ISSN: 1524-4571
- GOUMANS MARIE-JOSE ET AL: "Human cardiac progenitor cells are able to differentiate into cardiomyocytes in vitro" CIRCULATION, vol. 112, no. 17, Suppl. S, October 2005 (2005-10), page U106, XP008090751 & 78TH ANNUAL SCIENTIFIC SESSION OF THE AMERICAN-HEART-ASSOCIATION; DALLAS, TX, USA; NOVEMBER 13 -16, 2005 ISSN: 0009-7322
- MUMMERY C ET AL: "Differentiation of human embryonic stem cells to cardiomyocytes: Role of coculture with visceral endoderm-like cells", CIRCULATION, LIPPINCOTT WILLIAMS & WILKINS, US, vol. 107, no. 21, 3 June 2003 (2003-06-03) , pages 2733-2740, XP008029536, ISSN: 0009-7322, DOI: 10.1161/01.CIR.0000068356.38592.68
- HARDING ET AL: "The human embryonic stem cell-derived cardiomyocyte as a pharmacological model", PHARMACOLOGY AND THERAPEUTICS, ELSEVIER, GB, vol. 113, no. 2, 26 January 2007 (2007-01-26), pages 341-353, XP005738011, ISSN: 0163-7258, DOI: 10.1016/J.PHARMTHERA.2006.08.008
- VAN DER HEYDEN MARCEL A G ET AL: "Twenty one years of P19 cells: what an embryonal carcinoma cell line taught us about cardiomyocyte differentiation.", CARDIOVASCULAR RESEARCH 1 MAY 2003, vol. 58, no. 2, 1 May 2003 (2003-05-01), pages 292-302, ISSN: 0008-6363

## Description

The present invention relates to cardiomyocyte progenitor cells (CMPCs), preferably human CMPCs (hCMPCs). Methods for the isolation of such cells are also provided. The present invention also relates to the use of the CMPCs for the provision of cardiomyocytes, by way of differentiating the obtained CMPCs with a demethylating agent. Pharmaceutical compositions comprising these CMPCs for use in a cardiomyocyte replacement therapy and/or for the treatment of myocardial infarction or for ameliorating the effects of myocardial infarction are also provided. These compositions can be used in cardiomyocyte replacement therapies and/or for the treatment of myocardial infarction or for ameliorating the effects of myocardial infarction. In a further aspect, the present invention relates to the use of the CMPs obtainable by the methods of the invention in screening methods, e.g. drug screening methods.

If not defined otherwise, the terms used herein have the meaning and scope that they normally have in the relevant art of medicine or biochemistry, particularly in the field of cardiology, immunology, cell biology, electrophysiology and molecular biology.

Myocardial infarction is a common, severe cardiovascular disorder and a major cause of heart failure and cardiac death. Poor tissue perfusion and defective cardiomyocyte function are underlying mechanisms for the development of myocardial dysfunction, and the inability of remaining cardiomyocytes to regenerate and compensate the loss of ventricular cell mass. Stem cells have been studied intensively as a source of new cardiomyocytes to ameliorate injured myocardium and improve cardiac function ¹⁻⁴. The potential therapeutic benefit of stem cell transplantation has been investigated in animal models using bone marrow cells ⁵⁻⁷, cardiac stem cells ⁸, embryonic stem (ES) cells ^{9,10} and foetal cardiomyocytes ^{11,12} injected at the site of cardiac injury. The results reported in these animal studies has already let to the initiation of several clinical trials although at present only using bone marrow derived cells and skeletal myoblasts have been assessed in clinical trials ¹³⁻¹⁶. The developmental plasticity of bone marrow cells to differentiate into cardiomyocytes has been questioned ^{17.18} and the predominant *in vivo* effect of bone marrow or endothelial progenitor cells may be neoangiogenesis but not muscle regeneration. Autologous transplantation of skeletal myoblasts is confounded by the induction of life-threatening arrhythmias despite partial integration, survival and contribution to cardiac contractility. Another source of transplantable cardiomyocytes are cardiomyocytes derived from human embryonic stem cells (hES) cells. Although hES cells can be directed into the cardiomyocyte lineage, with a fetal phenotype ¹⁹, differentiation is not homogenous despite recent improvements in protocols ^{20,21}. Furthermore, immunogenic, arrhythmogenic and ethical problems will limit their clinical use.

Accordingly, the technical problem underlying the present invention was the provision of means and methods for cardiomyocyte replacement therapies.

This technical problem is solved by the provision of the embodiments as characterized in the claims.

Recently, different cardiac stem cell populations have been identified in the heart ², namely, cells expressing stem cell factor receptor (c-Kit ⁸), stem cell antigen-1 (Sca-1 ²²), or homeodomain transcription factor (islet-1 ²³) on their cell surface, side population cells (SP ²⁴) and cells able to grow in cardiospheres ²⁵.

Although rodent cardiac cell populations have been shown to be capable of differentiation into cardiomyocytes, either in vitro or in vivo, there has been no undisputed evidence to date, that any of the human cardiac stem cell populations are able to differentiate into functional mature cardiomyocytes. The different human cardiac stem cells populations isolated so far, only differentiated into cardiomyocytes in vitro when co-cultured with neonatal cardiomyocytes.

We isolated human cardiomyocyte progenitor cells (hCMPCs) from human heart tissue using an anti-Sca-1 antibody (Goumans et al., 2005. Circulation 112: U106), although a bonafide Sca-1 determinant in human cells had been disputed. The cells we selected using a Sca-1 antibody from both fetal and adult human heart proved amenable to expansion in culture. The progenitor cell population isolated from the human heart were defined as immunophenotypically distinct from previously described cardiac stem and progenitor cells (reviewed in ¹⁻³). Most remarkable in our study was the isolation and culture of hCMPCs from atrial biopsies of adult patients undergoing cardiac surgery, in particular coronary artery bypass graft (CABG). The unexpectedly high frequency with which this was possible opens perspectives, e.g. for autologous transplantation at a later date then the initial surgery if cultures were carried out under clinically compatible conditions.

Several groups have isolated cell populations from the rodent and human heart ⁸,²²⁻²⁵and all of these cardiac-derived stem cell populations are distinct from the CMPCs disclosed herein, based on their origin (i.e. the part of the heart they were isolated from), their gene expression profile, their expression profile of cell surface markers and their growth characteristics. Significant differences are partly summarized in the following Table 2 and are further explained herein below.

**Table 2**

| | CMPC of the invention | c-kit | Isl-1 | Sca-1 (mouse) |
|---|---|---|---|---|
| Marker expression | Sca-1 +/c-kit+/-/CD31 + | Sca-1 +/c-kit+/CD31- | Sca-1-/c-kit-/CD31- | Sca-1+/c-kit-/CD31- |
| | Nkx2.5+/Gata4+/Mef2c +/(prior to differentiation) | Nkx2.5+/Gata4+/Mef2c+ (only when grown in diff medium) | Nkx2.5+/Gata4+ | Nkx2.5- |
| | Isl1+ | Isl1- | lsl1+ | |
| | CD34-/CD45- | CD34-/CD45- | ND | |
| Isolation | Sca-1 expression | C-kit expression | co-culture with cardiac fibroblasts results in the formation of Isl1+ clusters | Sca-1 expression |
| Growth | Adherent cells when cultured on gelatin coated dish | Non adherent fraction replated | co-culture with cardiac fibroblasts | |
| | Auricle of the heart; | | | |
| Location | Adult human heart in atria Foetal heart in atria, AV boundary and within the ventricle | Human ventricle | Human post-natal heart, atria ventricle and OFT | Mouse heart |

An apparent difference which can be observed between the cells described in the art and the isolated cells (CMPCs) underlying the present invention is the expression of Sca-1 epitope, (a term which will be explained herein below) and CD31 on the cell surface.

The present invention, thus, relates in general to isolated cardiomyocyte progenitor cells (CMPCs) which are characterized by Sca-1 epitope and CD31 on their cell surface. "Isolated" refers to material removed from its original natural environment, and thus is altered "by the hand of man" from its natural state.

Furthermore, we provide the first evidence for functional differentiation of human myocardial derived progenitor cells into cardiomyocytes. Messina et al ²⁵ stimulated explants grown from cardiac biopsies with cardiotropin and generated cardiospheres. While mouse cardiospheres were spontaneously started to beat, with primitive cells at the inside and beating myocytes at the edges, human cardiospheres only differentiated when co-cultured with rat cardiomyocytes Isolated mouse Isl-I⁺ progenitor cells differentiated into cardiomyocytes when co-cultured with neonatal cardiomyocytes ²³, as was also shown for mouse cardiac SP cells ³².

The cardiomyocyte progenitor cells (CMPCs) as described herein, are capable of differentiation into cardiomyocytes in the absence of co-cultured neonatal cardiomyocytes, for example in the absence of neonatal rat cardiomyocytes.

The term "in the absence of co-cultured neonatal cardiomyocytes" means that the isolated human cardiomyocyte progenitor cells (hCMPCs) have not to be cultivated in the presence of exogenously added cardiomyocytes as described in the art, to differentiate in cardiomyocytes. The "absence" however does not exclude that small portions of endogenous cardiomyocytes are still present. Small portions in this regard includes that up to about 5, 4, 3, 2, 1 or 0,5% of the total cell population as isolated by the methods of the invention as described herein, consist of endogenous cardiomyocytes, i.e. those which were isolated together with the envisaged CMPCs. These co-isolated endogenous cardiomyocytes, however, are regularly absent after about 2 passages. In order to exclude a contamination with co-isolated cardiomyocytes, it is normally sufficient to use a CPMC passage > 8. To the more, it is preferred that the CMPCs which were isolated for example by the methods of the invention normally contain < 5, 4, 3, 2, 1, or 0.5% contamination with other cell types, for example with fibroblasts. The quality of the CMPCs may be observed by standard staining methods in order to exclude contamination with, for example fibroblasts, if desired.

The CMPCs of human origin.

"Human cardiomyocyte progenitor cells (hCMPCs)" are cells which can be characterised as follows:
(A) Morphologically, hCMPCs are less than (about) 40 µm in diameter and exhibit a high nucleus-to-cytoplasm ratio. "High" nucleus to cytoplasm ration means in this regard that at least about 50, 60, 70 or 80% of the volume of the cell is filled by the nucleus. Early isolated cells (i.e. cells at early passages, for example passage 5) are round/cuboidal cells that will elongate to a small fibroblast shape.
(B) Growth characteristics: hCMPCs can be clonally expanded and, after an initial lag phase of (about) 3-10 days, divide (about) once every 18-26 hours. When cultured on gelatine coated dishes, hCMPCs grow as adherent cultures.
(C) Biochemistry: Immunology:
   (a) they lack CDH b₁ CD13, CD14, CD29, CD34, CD45, CD68, CD71 , CD133, SSEA-3 and SSEA-4 when determined in FACS analysis; "lack"
      means that no significant levels are detectable in a standard FACS
      means that no significant levels are detectable in a standard FACS analysis
   (b) they express Sca-1, CD105, CD31, CD59 and low levels of c-kit when determined in FACS analysis;
   (c) they express Islet-1 (Isl-1), Nkx2.5, Gata-4 and low levels of mef2c in RT-PCR (for example when using the primers as shown in Table 1 in the appended examples and when using the PCR-protocol exemplified in Example 3)
   (d) they lack the expression of endothelial-, smooth muscle cell-, or cardiomyocyte specific genes (VE-cadherin, Von Willebrand factor for endothelial cells; Desmin or smooth muscle cell heavy chain myosin for smooth muscle cells and cardiac actin, MLC2v for cardiomyocytes). "Lack the expression" means in this regard that the cells do not express significant levels of the genes when evaluated with Blotting or micro array techniques.
   (e) they express the potassium inward rectifiers Kir2.1 and 2.2 as determined by RT-PCR. Kir2.x modulates the Ik1 current, involved in late repolarization and important for stabilizing the resting membrane potential of cardiomyocytes. Immature cardiomyocytes often lack, or have low Ik1 currents, resulting in higher resting membrane potentials, shorter action potentials, and increased excitability.
   (f) they express latent TGFβ binding protein 4 (mRNA e.g. in RT-PCR and protein by western blotting), Fibroblast growth factor receptor 4 (mRNA e.g. in RT-PCR and protein by western blotting), and Vascular endothelial growth factor receptor 2 (mRNA e.g. in RT-PCR and protein by FACS).

"Cardiomyocytes" are cells which are, inter alia, characterised by the formation of cross striations, the expression of sarcomeric proteins (α-actinin, Troponin I, β-MHC, titin, desmin, MLC-2V), and atrial natriuretic factor (ANF). Cardiomyocytes are furthermore characterised by the expression of connexins on the cell membrane, by by excitation-contraction coupling. Cardiomyocytes normally have a diameter of over 120 µm.

The cardiomyocytes disclosed herein (i.e. those derived from the human CMPCs and/or obtainable by the methods of the invention) are furthermore characterized by the following technical features which makes them unique in comparison to cardiomyocytes disclosed in the art. They are more mature then the stem cell derived cardiomcytes described so far, based on their low maximal diastolic membrane potential which is much lower than the ones from human embryonic stem cells or mouse Isl-1 derived cardiomyocytes. The maximum diastolic or resting membrane potential is caused by the difference in ionic charge across the membrane of the cell during phase 4 of the action potential. The normal resting membrane potential in the adult ventricular myocardium is about -85 to -95 mV. This potential is determined by the selective permeability of the cell membrane to various ions. Up to date, the only other reported resting membrane potential from human stem cell derived cardiomyocyte is those from embryonic stem cells.

Embryonic stem cell derived cardiomyocytes have a maximal diastolic membrane potential of about -48 mV, while CMPC derived cardiomyocytes have a maximal resting membrane potential of about -67 mV or lower.

The present description further relates to the cardiomyocytes as described herein. These cardiomyocytes are obtainable by differentiating the

CMPCs by way of the methods described herein. These cardiomyocytes can be comprised in a pharmaceutical and/or diagnostic composition which will be exemplified in more detail below.

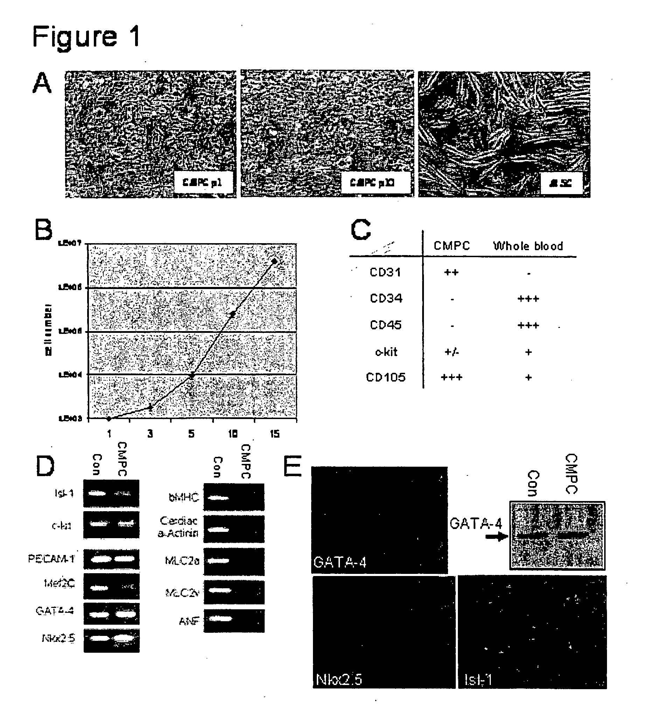

To isolate CMPCs from heart tissue (fetal or adult), atrial biopsies, e.g. derived from the auricle of the heart were cut into small pieces which were subsequently incubated in collagenase/protease solution in PBS with 0.5% serum either 2 hours at 37 degrees centigrade or overnight at 4 degrees centigrade. This was followed by differential centrifugation (50 g for 5 min) to separate the large cardiomyocytes from the small non-myocyte fractions in which the CMPCs are resident. The collagenase that can be used in this regard is for example type I from SIGMA (1 mg/ml) or Worthington Type II (0.5 mg/ml). This procedure resulted in a resident. The collagenase that can be used in this regard is for example type I from SIGMA (1 mg/ml) or Worthington Type II (0.5 mg/ml). This procedure resulted in a nucleated cell suspension virtually depleted of cardiomyocytes. Using an anti Sca-1 antibody coupled to magnetic beads (obtainable e.g. from Miltenyi Biotec, Sunnyvale, CA) a cell fraction with a diameter of about <50 µm and high growth potential was isolated by magnetic cell sorting (MACS, Miltenyi Biotec, Sunnyvale, CA), following the manufactures protocol. (Fig. 1b). Sca-1 positive cells were eluted from the column by washing with PBS supplemented with 2% fetal calf serum (FCS) and cultured on 0.1% gelatin coated dishes in M199 (Gibco)/EGM (3:1) supplemented with 10% FCS (Gibco), 10 ng/ml basic Fibroblast growth factor (bFGF), 5 ng/ml epithelial growth factor (EGF), 5 ng/ml insulin like growth factor (IGF-1) and 5 ng/ml hepatocyte growth factor (HGF). After a lag phase of 3 days, the cells started to proliferate and colonies formed from small spindle-shaped cells with a high nucleus-to-cytoplasm ratio (Fig. 1a). hCMPCs could be clonally expanded. After limited dilution, single cells grown in a 96-well plate generated colonies with a 5-25% efficiency.

We have also used the following methods to isolate CMPCs:
(a) the nucleated cell suspension virtually depleted of cardiomyocytes were incubated with Sca-1 antibody from R&D (according to the manufacturers instructions) and we came back with rat secondary antibody coupled on magnetic beads to isolate Sca-1 positive cells; or
(b) we have also used a Sca-1 antibody labelled with FITC and either FACS-sorted the cells, or used an anti-FITC antibody coupled to magnetic beads to isolate Sca-1 positive cells.

"Sca-1 antibodies" that can be used in accordance with the present invention are exemplified in the following but it has to be understood that the methods of the invention are not limited to these specific antibodies:
Rat monoclonal Sca1 antibody clone D7(Abcam), Rat-anti-Mouse Ly6a/e clone D7 (cederlane, eBioscience), Rat monoclonal Sca-1 clone 177228 (R&D system), Anti-Mouse Sca-1 (Ly-6A / E) Monoclonal Antibody, Clone 38/42 Leinco Technologies (Rat Anti-Ly-6A / E Monoclonal Antibody, Clone E13-161.7 (Pharmingen, Stemcell technologies). These antibodies can either be not conjugated, or conjugated to biotin, FITC, TRITC or other well-known labels known in this field (Immunology) and labels to that a secondary isolation step can be performed to. Such labels and secondary isolation steps are mentioned herein and are also well-known to the skilled person. It is, for example possible to use an FITC-labelled Sca-1 antibody. The Sca-1 positive cells are thus sortable, e.g., by FACS. The explanations given herein equally apply to other antibodies which might be used alternatively or in combination with Sca-1 , e.g., CD31 antibodies or functional fragments thereof. It is thus also possible to sort the CMPCs with CD31 antibodies or a combination of CD31 and Sca-1 antibodies.

The present invention relates in a further aspect to a method for the enrichment of cardiomyocyte progenitor cells (e.g. human CMPCs), said method comprising or essentially consisting of the steps of:
(a) dissociate heart tissue e.g. derived from the atrium; and
(b) enrichment of CMPCs with a Sca-1 binding agent and a CD31 binding agent.

"Heart tissue" includes all parts of the heart which comprise the CMPCs. Said heart tissue preferably is derived from the atrium or parts of the atrium of the heart, e.g. the auricle. The term "auricle" relates to a small conical pouch that projects from each atrium of the heart.

The dissociation step of the heart tissue as described herein elsewhere, which is preferably enzymatically but can also be mechanically, is optionally followed by a differential centrifugation step or comparable methods like for example a centrifugation in a Percoll gradient. Such methods are well known to the skilled person and are explained in detail in standard laboratory handbooks like Sambrook et al.. A differential centrifugation or a comparable method causes the separation of mixtures such as cellular particles in a medium at various centrifugal forces to separate particles of different density, size, and shape from each other. Such a step will result in a nucleated cell suspension virtually depleted of cardiomyocytes. The differential centrifugation which may be used in the methods of the invention could be exemplarily performed with a force of about 50 g for about 5 min. "Virtually depleted" means in this regard that small portions of other cells, like cardiomyocytes, can be present in the non-myocyte fraction, where the CMPCs are also present. Small portions in this regard includes that up to about < 5, 4, 3, 2, 1 , or 0.5% of the total cell population as isolated by the methods of the invention consists of other cells, i.e. not the envisaged CMPCs. Following the separation, CMPCs will be sorted from the non-myocyte fraction. As mentioned earlier, it is preferred that the CMPCs which were isolated for example by the methods of the invention normally contain < 5, 4, 3, 2, 1 , or 0.5% contamination with other cell types, for example with fibroblasts. The quality of the CMPCs may be observed by standard staining methods in order to exclude contamination with, for example fibroblasts, if desired.

Methods to dissociate heart tissue, for example the auricle of the heart, either enzymatically or mechanically, are well-known to the skilled reader and to the more exemplified in the appended examples.

In preferred methods, enzymatically dissociation is used, and the enzyme is preferably a collagenase although other proteases might also be employed. The tissue can for example also be dissociated with a combination of Collagenase (Worthington) and Pancreatine (Alkemi A0585,0100 Applichem). The Sca-1 epitope is, however, sensitive for trypsin and thus trypsin should be avoided if it is intended to isolate the cells with anti-Sca-1 binding agents as described herein elsewhere. It will be understood, however, that other enzymatically dissociation methods are also known which may be used alternatively for example based on the manufacturer's instructions and/or based on standard laboratory books. Protocols for tissue dissociations are known in the art and frequently published for example in laboratory handbooks. These dissociation-methods are also included as long as the methods result in dissociated cell suspensions, which comprise the cardiomyocyte progenitor cells (CMPCs).

"Sca-1" is a mouse-antigen, particularly an 18 kDa phosphatidylinositol-anchored protein which is a member of the Ly-6 family of GPI-linked surface proteins. The Ly-6 family is involved in regulation and function of T cell activation. Sca-1 is a major phenotypic marker for mouse hematopoietic progenitor/stem cell (HSC) and has been used as a marker of HSC in mice of both Ly-6A/E haplotypes. The corresponding "human" counterpart of Sca-1 is not yet identified. A "Sca-1 -like epitope" thus relates to orthologs or analogs of the mouse Sca-1. "Orthologs" have evolved from a common ancestral gene by specification and thus relate to the "same" protein in a different species, while "analogs" refers to two proteins that have the same or similar function, but that have evolved separately. Normally, orthologs or analogs of mouse Sca-1 are polypeptides which have the same or similar functions but differ from the mouse Sca-1 e.g. by post-translational modifications, by amino acid sequence differences, or by both. Thus, cardiomyocyte progenitor cells (hCMPCs) which have an epitope, i.e. a "Sca-1 like epitope" on their surface which is characterized by its ability to specifically react with Sca-1 antibodies, preferably those exemplified above, in such a way that it is at least possible to sort these "Sca-1" positive cells by routine sorting methods also described herein (for example MACS). It has to be understood that "Sca-1" on cells, (besides mouse cells), is used herein interchangeably with "Sca-1 epitope" on these cells (besides mouse cells which express the Sca-1 epitope).

A "Sca-1 binding agent" is preferably an antibody or a functional fragment thereof, which is capable of binding to the Sca-1 epitope described herein. The "binding" in this regard means that the Sca-1 positive cells are bound by the binding agent in such a way that the cells can be subsequently sorted by means and methods well known in the art and also described herein. The minimum requirement of a "binding agent" as used herein is, thus, that it is able to bind specifically to Sca-1 positive cells and/or cells expressing a Sca-1 like epitope and allows for the sorting or separation of these cells. The term "specifically binding" in connection with the binding agent used in accordance with the present invention means that the binding agent, e.g. the antibody etc. does not or essentially does not cross-react with other polypeptides. Antibodies that bind to the Sca-1 epitope but do not or do not essentially bind to other polypeptides on the cell surface are considered specific and are, therefore, preferably selected for the methods of the invention.

Said CMPCs are preferably enriched with a Sca-1 binding agent. In a particularly preferred embodiment of the method of the invention, said binding agent is an anti-Sca-1 antibody or a functional fragment thereof.

The term "enriched" means that the relative number of the Sca-1 positive cells is increased in the cell suspension after the cell sorting when compared to the other cells (e.g. cardiomyocytes) which were also contained in the initial cell suspension. Preferably, the CMPCs are enriched, i.e. their relative cell number is increased to at least 50, 60, 70, 80, 90, 95% or even more. Protocols for such an "enrichment" are exemplified herein, for example the methods to isolate CMPCs as described herein.

As described herein above, the cardiomyocyte progenitor cells (CMPCs) are characterized by Sca-1 epitope and CD31 on their cell surface. Thus, it is also envisaged that the CMPCs are enriched with a CD31 binding agent, e.g. an CD31 specific antibody or functional fragments thereof. The explanations given in the section above for example those which relate to the scope and meaning of "binding agent" and "functional fragments of antibodies" and "specific binding" equally apply to the CD31 marker which is also detectable on the CMPCs. To the more, each marker which is present on the surface of the CMPCs (e.g. CD105) might be used alternatively or in combination to the afore mentioned ones (CD31 and Sca-1) to enrich CMPCs, provided that this enrichment results in a cell fraction wherein the relative cell number of the CMPCs as described herein is increased to at least 50, 60, 70, 80, 90, 95% or even more.

In a further embodiment, it is also envisaged to enrich the cells with a Sca-1 and an CD31 binding agent, e.g. antibodies directed to these epitopes. Means and methods to detect such bound antibodies or fragments thereof are well-known and also described herein.

Anti-Sca-1 antibodies are well known in the art and also exemplified above. The list of these specific Sca-1 antibodies is however exemplarily and in no way limits the group of Sca-1 antibodies which might be employed when carrying out the methods of the invention.

CD31 antibodies are also well-known and exemplified by mouse α-CD31 monoclonal antibody clone WM59 (BD biosciences), α-human CD31 clone 1 F11 (Beckman coulter), mouse α-CD31 clone 158-2B3 (Cell signaling), α-CD31 clone HC1/6 (Chemicon), mouse α-human CD31 clone 9G11 (R&D systems).

CD105 antibodies are also well-known and exemplified by mouse α-CD105 clone 35 or clone 266 (BD biosciences), Goat α-human CD105 polyclonal antibody (R&D systems), mouse α-human CD105 clone 166707 of clone 166713 (R&D systems), α-CD105 clone 8E11 or clone P3D1 (Chemicon).

Other antibodies which are specific for Sca-1 or a Sca-1 like epitope or CD31 or CD105 or other epitopes useful in the described methods can be easily produced by methods well-known in the art. For example it is possible to use cell lines secreting antibodies to essentially any desired substance that produces an immune response. RNA encoding the light and heavy chains of the immunoglobulin can then be obtained from the cytoplasm of the hybridoma. The 5' end portion of the mRNA can be used to prepare cDNA to be inserted into an expression vector. The DNA encoding the antibody or its immunoglobulin chains can subsequently be expressed in cells, preferably mammalian cells. Depending on the host cell, renaturation techniques may be required to attain proper conformation of the antibody. If necessary, point substitutions seeking to optimize binding or stability of the antibody may be made in the DNA using conventional cassette mutagenesis or other protein engineering methodology such as is disclosed herein. Furthermore, antibodies or fragments thereof to the aforementioned epitopes (Sca-1 ) can be obtained by using methods which are described, e.g., in Harlow and Lane "Antibodies, A Laboratory Manual", CSH Press, Cold Spring Harbor, 1988. Other suitable antibodies as well as methods for testing the effectiveness of such antibodies are detailed in WO02071061.

For the production of antibodies in experimental animals, various hosts including goats, rabbits, rats, mice, and others, may be immunized by injection with polypeptides or any fragment or oligopeptide or derivative thereof which has immunogenic properties. Techniques for producing and processing polyclonal antibodies are known in the art and are described in, among others, Mayer and Walker, eds., "Immunochemical Methods in Cell and Molecular Biology", Academic Press, London (1987). Polyclonal antibodies also may be obtained from an animal, preferably a mammal. Methods for purifying antibodies are known in the art and comprise, for example, immunoaffinity chromatography. Depending on the host species, various adjuvants or immunological carriers may be used to increase immunological responses. When derivatives of said antibodies are obtained by the phage display technique, surface plasmon resonance as employed in the BIAcore system can be used to increase the efficiency of phage antibodies which bind to an epitope of the peptide or polypeptide (Schier, Human Antibodies Hybridomas 7 (1996), 97-105; Malmborg, J. Immunol. Methods 183 (1995), 7-13). In many cases, the binding phenomena of antibodies to antigens is equivalent to other ligand/anti-ligand binding.

The production of chimeric antibodies is described, for example, in WO89/09622. A further source of antibodies to be utilized in accordance with the methods of the present invention are so-called xenogenic antibodies. The general principle for the production of xenogenic antibodies such as human antibodies in mice is described in, e.g., WO 91/10741 , WO 94/02602, WO 96/34096 and WO 96/33735. The production of recombinant antibodies is described, for example, in R. Kontermann, S. Dübel: Antibody Engineering, Springer Lab Manual 2001. The antibody which is used in accordance with the uses or methods of the invention may be a monoclonal or a polyclonal antibody (see Harlow and Lane, "Antibodies, A Laboratory Manual", CSH Press, Cold Spring Harbor, USA, 1988) or a derivative of said antibody which retains or essentially retains its binding specificity. Preferred derivatives of such antibodies are chimeric antibodies comprising, for example, a mouse or rat variable region and a human constant region.

The term "functional fragment" as used herein refers to fragments of the antibodies as specified herein which retain or essentially retain the binding specificity of the antibodies like, separated light and heavy chains, Fab, Fab/c, Fv, Fab', F(ab')2. The term "antibody" also comprises bifunctional antibodies and antibody constructs, like single chain Fvs (scFv) or antibody-fusion proteins. The term "scFv fragment" (single-chain Fv fragment) is well understood in the art and preferred due to its small size and the possibility to recombinantly produce such fragments.

The binding agent or antibody etc. may be affixed to a carrier, for example to magnetic beads, column material or solid carriers like polystyrene plates or the like, which makes it possible to separate cells which specifically bind to the so affixed binding agents. Means and methods in this regard are well-known to the skilled person and furthermore explained in the appended examples.

The CMPCs are preferably enriched by MACS.

"MACS" means magnetic activated cell sorting and is a well-known and reliable method for the separation of cells according to specific cell surface markers. Cells stained sequentially e.g. with biotinylated antibodies, fluorochrome-conjugated avidin, and superparamagnetic biotinylated-microparticles (about 100 nm diameter) are separated on high gradient magnetic (HGM) columns. Unlabelled cells pass through the column, while labelled cells are retained. The retained cells can be easily eluted. For example the Anti-Sca-1 MicroBead Kit provided by Miltenyi Biotec includes all reagents for fluorescent and indirect magnetic labelling of Sca-1 + cells, using Anti-Sca-1-FITC and Anti-FITC MicroBeads. This Kit was used in the appended examples, but it will be understood that also other MACS-systems are at hand which might also be used.

As an alternative, the CMPCs are enriched by FACS. "FACS" means fluorescent-activated cell sorting which is a well- known method. As cells or particles pass through the FACS-instrument they can be selectively charged, based on user defined parameters, and can be deflected into separate paths of flow directed to different collection tubes. It is therefore possible to separate defined populations of cells from an original mix with a high degree of accuracy and speed by way of antibody-labeling of the cell suspension.

It will be understood that the other enrichment-methods are also contemplated, e.g. Enzyme-Linked Immunosorbent (ELISA)-techniques or columns with antibodies affixed thereto etc.

In a further embodiment, the present invention relates to a cell fraction comprising at least 90% of human CMPCs as defined herein, which are obtainable or obtained by the method(s) as described herein.

To phenotypically characterize the isolated CMPCs which were enriched by the methods disclosed herein, freshly isolated cells were examined for cell surface expression of haematopoietic markers (CD45 and CD34), stem cell marker (c-Kit), CD105 and CD31 (Pecam-1) by FACS analysis. To this end several antibodies were used which are detailed herein in the examples. Such antibodies are well-known to the skilled reader (see for example the catalogues and WebPages of the respective manufacturers of such antibodies for example Beckmann Coulter) but might be replaced by other antibodies which are also able to detect the mentioned epitopes. The isolated CMPCs were negative for CD34, CD45, but exhibited expression of CD31 , CD105 and moderate levels of c-kit (Fig. 1 c) when tested under the experimental conditions exemplified in the appended examples. RT-PCR analysis of the cells revealed that they do not express Oct4, a marker for pluripotent ES cells. These results indicated that the cardiac derived progenitor cells disclosed herein do not belong to the haematopoietic stem cell or previously described cardiac stem cell population.

To further characterize CMPCs, the expression of genes specific for early cardiac development was examined by RT-PCR using cultured cells that had been passaged 8-10 times (Fig. 1d) and using the primers as listed in Table 1. As can be seen in figure 1d, cultured hCMPCs express c-Kit and Islet-1 as well as GATA-4, Nkx2.5 and Mef2C, suggesting that they are more restricted to the cardiac lineage. Besides mRNA, CMPCs also expressed GATA-4, Nkx2.5 and Islet-1 protein (Fig. 1e), although they did not express cardiac genes, like bMHC, α-actinin, ANF, MLC2A or MLC2V (Fig. 1d). Interestingly, the subcloned hCMPCs showed the same characteristics as the nonclone-derived CMPCs.

Also adult hCMPCs were isolated from enzymatically dissociated atrial biopsies, using an anti Sca-1 antibody and the methods described hereinbefore. Seeding these cells on gelatin-coated dishes in culture medium resulted in growing progenitor colonies in 24 out of 33 cases. Adult hCMPCs had the same stem-cell-like morphology with a large nucleus and little cytoplasm; no loss of proliferation potential was noted after 23 passages. Three to 6 weeks after isolation adequate numbers of cells were obtained for further analysis. FACS-analysis revealed that cultured adult hCMPCs were CD34 negative/CD45negative and 5% of the cells expressed c-kit. The gene-expression profile was analyzed by RT-PCR and revealed that adult hCMPCs had the same expression profile as fetal derived hCMPCs (Fig. 1). Immunofluorescent staining revealed that almost all adult cells were Islet-1 \ although predominantly cytoplasmic.

We have also established differentiation of CMPCs into tubular vascular structures (angiogenesis) on Matrigel, said "tubular vascular structures" which are sometimes also denoted herein as "tubular structures" containing aligned endothelial cells encircled by smooth muscle cells. The formation of these newly formed capillary tubes (i.e. the "tubular structures" containing aligned endothelial cells encircled by smooth muscle cells) can be induced by the addition of VEGF (see Example 8). The formation of these newly formed capillary tubes can be visualised for example by staining α-SMA (α smooth muscle actin) on smooth muscle cells and/or PECAM-1 (platelet endothelial cell adhesion molecule-1, CD31) on endothelial cells (see also appended Example 8). Antibodies which can be used in this regard are exemplified in the follwoing: SMA antibody: AB5694 from abcam, clone 1A4, Sigma, cat. Number: A2547, or Dako, cat. Number M08510. Endothelial cells: PECAM-1 clone 9G11 or CD34 clone Qbend/10 form Biogenex or CD31 from Sigma and ve-cadherin from Santa Cruz.

Angiogenesis is the generation and formation of new capillary blood vessels, a process fundamental for processes like wound healing and reproduction. It's also involved in pathological processes (rheumatoid arthritis, tumor growth and metastasis).

"Matrigel" is the trade name for a gelatinous protein mixture secreted by mouse tumor cells and marketed by BD Biosciences. This mixture resembles the complex extracellular environment found in many tissues and is used as a substrate for cell culture.

The description further relates to an in vitro method for the differentiation of CMPCs into tubular structures or vascular sprouts comprising the steps of:
(a) providing the CMPCs, preferably on Matrigel and(or any other gelatinous protein mixture which resembles the complex extracellular environment; and
(b) optionally treating said CMPCs with an biologically active agent having the capabilities to induce angiogenesis or sprouting angiogenesis (like e.g. VEGF).

According to the description, it is preferred that the CMPCs to be differentiated in the above in vitro method for the differentiation of CMPCs into tubular structures or vascular sprouts are seeded on Matrigel and/or any other gelatinous protein mixture which resembles the complex extracellular environment.

The present description relates to the "tubular structures" or vascular sprouts as described herein. These "tubular structures" or "vascular sprouts" are obtainable by differentiating the CMPCs by way of the methods described hereinabove. These "tubular structures" or "vascular sprouts" can be comprised in the pharmaceutical and/or diagnostic compositions of the present description and may be furthermore used in the screening assays/methods of the present description.

In a further aspect, the use of the CMPCs defined herein for the provision of cardiomyocytes and/or tubular structures and/or vascular sprouts is described.

As described, the CMPCs are propagated/expanded in vitro (exemplified in Example 1 /culture conditions of the Sca-1 isolated cells) in order to further expand their cell number, before the CMPCs are actually differentiated into the cardiomyocytes or into tubular structures or vascular sprouts which were mentioned before.

We have also established differentiation of CMPCs into adipocytes. To this end, CMPCs are seeded and cultured until they have reached a confluence of 90 - 100% in SP++ (SP++: EBM-2 + EGM-2 single qouts Cat. No. CC-4176 + 2% FBS). Subsequently, the medium is replaced by DMEM 4.5g/l glucose + Na pyruvate containing 10% FBS, 1 uM dexamethasone, 0.5mM 3- Isobutyl-1-methylxanthine (IBMX), 10 ug/ml Insulin, 0.2 mM indomethacin and Pen/strep. After some days, droplets of fat are deposited around the nucleus. Every 3 days the medium is refreshed.

The present description thus relates in a further aspect to an in vitro method for the differentiation of CMPCs into adipocytes comprising the steps of:
(a) providing the CMPCs, and
(b) treating said CMPCs with IBMX, indomethacin and insulin.

For a cell type to be suitable for cardiomyocyte replacement therapies, it should not only be able to proliferate but also differentiate efficiently into cardiomyocytes. To study the capacity of the hCMPCs to differentiate into cardiomyocytes, cells were plated at 10⁵ per cm² in differentiation medium containing ascorbic acid and treated with 5-azacytidine (which is a demethylating agent). Three weeks after treatment, hCMPCs formed elongated aggregates and spontaneously beating areas were observed. We thus demonstrated that hCMPCs are able to differentiate into mature cardiomyocytes in vitro after 5-azacytidine treatment in the presence of an antioxidant namely ascorbic acid, even after prolonged passage.

The present invention thus relates to an in vitro method for the differentiation of CMPCs into cardiomyocytes, comprising the steps of:
(a) providing a cell fraction comprising the CMPCs according to claim 1; and
(b) treating said CMPCs with a demethylating agent
(c) allowing the so-treated CMPCs to differentiate into cardiomyocytes.

As mentioned before, the CMPCs are surprisingly able to differentiate into cardiomyocytes even in the absence of co-cultured neonatal cardiomyocytes. It is thus preferred that these differentiation methods of the invention are carried out in the absence of co-cultured neonatal cardiomyocytes. It is also envisaged that all the differentiation methods described herein (cardiomyocytes and/or tubular structures) can be carried out in the absence of co-cultured neonatal cardiomyocytes.

DNA methylation is an epigenetical mechanism that plays crucial roles in cellular differentiation and tissue development in embryogenesis. A "demethylating agent" as used herein is an agent which is able to demethylate DNA, for example by way of inhibiting the intracellular DNA methyltransferase(s). Such agents are well-known to the skilled person and include for example 5-aza-cytidine or 5-aza-2'-deoxycytidine (5-aza), which are preferred, but the methods of the invention are not limited thereto.

In a preferred embodiment, said hCMPCs are treated with the demethylating agent mentioned above in the presence of an antioxidant agent, preferably ascorbic acid. An "antioxidant agent" in this regard refers to an anorganic or organic chemical that reduces the rate of oxidation reactions. Further examples for antioxidants which might be used in the context of the present invention are 2-mercapthoethanol, tocopherol or BHA but it will be understood that further antioxidant agents might be used as long as they exert the desired effect of cell differentiation described herein.

In a preferred embodiment, the present invention thus relates to a cell fraction comprising at least 90% of the CMPCs defined hereinabove, said cells being capable of differentiation into cardiomyocytes in vitro after 5-aza-cytidine or 5-aza-2'-deoxycytidine treatment in the presence of ascorbic acid.

Apart from antioxidant agents like ascorbic acid²⁷, TGFβ family members, including TGFβ and BMP, have also been found to promote cardiomyogenic differentiation in ES cells, and be critical for the expression of cardiac specific markers. ^{9,10,26,28}. Furthermore, TGFβ stimulated myogenic differentiation of c-kit+ bone marrow stem cells ²⁹. Therefore, we explored the capacity of TGFβ to improve cardiomyogenic differentiation of hCMPCs.

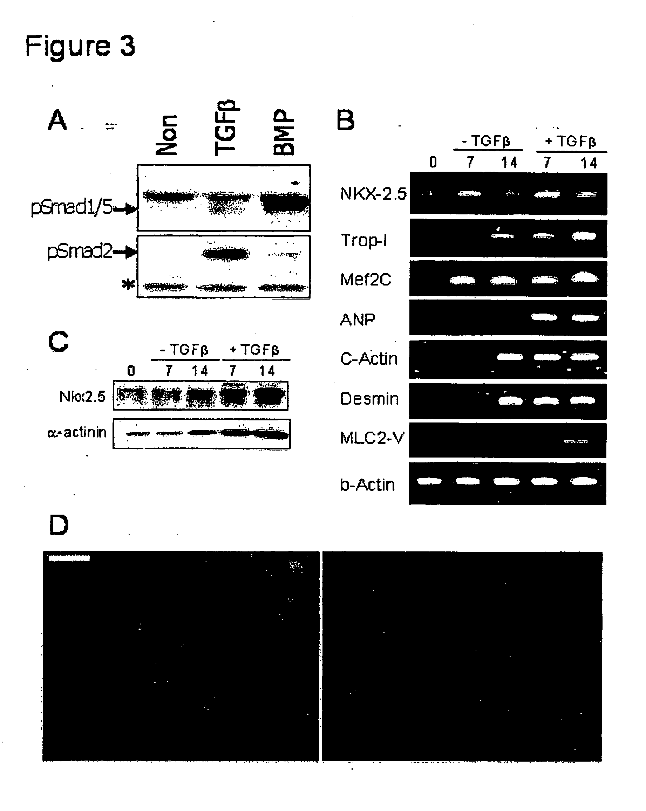

To determine whether CMPCs were capable of responding to TGFβ ligand we examined their ability to phosphorylate smad proteins. TGFβ exerts its effect by binding to a complex of type I and type II receptor and phosphorylating Smads ³⁰. TGFβ phosphorylates Smad2 while BMP phosphorylates Smad1. As shown in figure 3a CMPCs show clear BMP induced Smad1 phosphorylation as well as TGFβ induced Smad2 phosphorylation indicating that they express the appropriate TGFβ and BMP receptor combinations to respond to ligand stimulation. To examine the effect of TGFβ on cardiomyocyte differentiation, hCMPCs were plated at 2 x10⁴ cells per cm² in differentiation medium, treated with 5-aza for 24 hours and cultured for up to 3 weeks in differentiation medium supplemented with TGFβ (1 ng/ml). RT-PCR analysis showed that expression of several cardiac genes like Troponin-I, ANP, cardiac-actin and desmin was already observed 7 days after the initiation of differentiation in the presence of TGFβ, but these genes were undetectable in its absence (Fig. 3B). MLC2v was normally detected in cultures without TGFβ only after 3 weeks but was already present after two weeks in its presence. Western blot analysis showed that the expression of Nkx2.5 and α-actinin in CMPCs increased dramatically after 7 days of culture in the presence of TGFβ (Fig. 3c). Immunofluorescent staining demonstrated that the percentage of α-actinin positive cells increased to nearly 100% (Fig. 3d). Addition of an ALK5 kinase inhibitor to the cell cultures known to block TGFβ signaling ³¹, inhibited the differentiation of hCMPCs into cardiomyocytes (data not shown). These results indicated that TGFβ stimulation increases the differentiation of hCMPCs into cardiomyocytes to nearly 100%.

Accordingly, it is preferred that the in vitro methods for the differentiation of CMPCs into cardiomyocytes as defined herein further or alternatively comprise the step of treating the CMPCs with a TGF-β family member. TGFβ exists in at least three known subtypes in humans,TGF-β1 , TGF-β2 and TGF-β3. The TGFβ superfamily includes several proteins, for example TGF-β, activin and bone morphogenetic proteins (BMPs) BMP2 through BMP7. A member of the TGFβ-family thus includes any of the aforementioned substances either alone or in any desired combination. It is also envisaged to provide recombinant cells which express and secrete one or more members of the TGFβ-family instead of providing/adding the TGFβ-family member per se. Examples of recombinant cells include the CMPCs and/or cardiomyocytes or any other cell which is (a) able to express and secrete a member of the TGF-β family and (b) is biocompatible either with the CMPCs/cardiomyocytes and/or with the subject to be treated (see for example the description of the pharmaceutical compositions below).

The present invention thus relates to an in vitro method for the differentiation of CMPCs into cardiomyocytes, further comprising the steps of:
(b1) treating said CMPCs with a TGF-β family member.

It will be understood that the so-treated CMPCs are treated for a time which is sufficient to allow the CMPCs to differentiate into cardiomyocytes. The differentiation can be determined for example by immunofluorescence analysis of contractile protein expression, e.g. of α-actinin. Furthermore, it will be understood that if the cells start to beat, they are differentiated. The cross-striations can also be determined using light microscopy. It is also envisaged to modify the CMPCs using a cardiomyocyte specific promoter coupled to a detectable marker gene for example to GFP so that one can follow the differentiation of the cells more easily. Suitable cardiac promoters are for example human cardiac actin promoter and the Mlc-2V promoter.

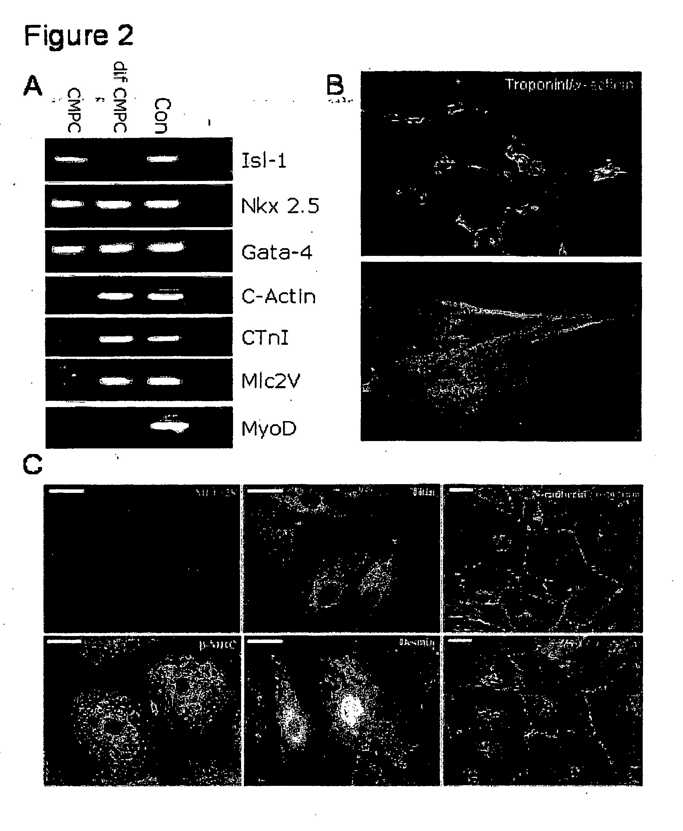

Four weeks after stimulation, mRNA expression of cardiac differentiation markers was analyzed by RT-PCR. Before differentiation, only GATA-4 and Nkx2.5 were expressed, while after 4 weeks of stimulation, hCMPCs-derived cardiomyocytes expressed the CM-specific genes bMHC, ANP, Troponin I and cardiac α-actin (Fig. 2a). Moreover, no Myo-D mRNA could be detected, indicating that there was no differentiation towards skeletal myocytes. To characterize the cardiomyocytes further, immmunofluorescent staining for sarcomeric proteins was carried out. hCMPC-derived cardiomyocytes exhibited sarcomeric striations when double stained with α-actinin and troponin-I, organized in separated bundles (Fig. 2b). Labeling of the cells with antibodies raised against other components of the contractile apparatus revealed a pattern of cross striations positive for titin, desmin, MLC2v, and β-MHC (Fig. 2c). Double labeling of α-actinin with the intercalated disk related proteins like N-cadherin or ZO-1 resulted in strong positive signals at the cellular boundaries of nearly all cardiomyocytes indicative of stable mechanical coupling (Fig. 2c).

The cardiomyocytes as described herein, i.e. those obtainable by the in vitro methods for the differentiation of CMPCs into cardiomyocytes as defined herein, are thus characterized by the expression of β-MHC, ANP, α-actinin, cardiac α-actin, Troponin I and/or MLC2v cardiac mRNA detected by RT-PCR and/or by the absence of Myo-D and/or Isl-1 mRNA detected by RT-PCR.

The cardiomyocytes disclosed herein furthermore are more mature then the ones described so far, based on their low maximal diastolic membrane potential which is much lower then the ones from human embryonic stem cells or mouse Isl-1 derived cardiomyocytes.

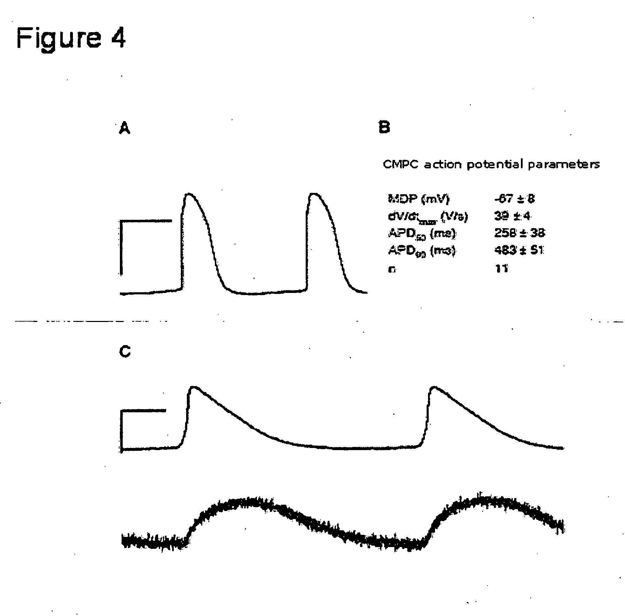

To further characterize the cardiomyocytes, we performed patch-clamp electrophysiology. Action potentials recorded from single cardiomyocytes and cardiomyocytes in small clusters (n=11) had a ventricle-like shape (Fig. 4A), as confirmed by quantification of action potential parameters (Fig. 4B). Average maximal diastolic potential was -67 ± 8 mV and average maximal upstroke velocity was 39 ± 4 V/s. Average APD₅₀ and APDg₀ were 258 ± 38 and 483 ± 51 ms, respectively. Furthermore, diastolic membrane potential was rather stable, unlike cardiac pacemaker cells. Excitation-contraction coupling was demonstrated using combined calcium-imaging and current clamp experiments (Fig. 4C). Average time to peak of the transients (n=7) was 447 ± 54 ms, time of half-inactivation was 568 ± 42 ms.

The cardiomyocytes as described, i.e. those obtainable by the in vitro methods for the differentiation of CMPCs into cardiomyocytes as defined herein, are thus further characterized by their ability to show excitation-contraction coupling. Excitation-contraction coupling (ECC) is the process by which an action potential triggers a myocyte to contract.

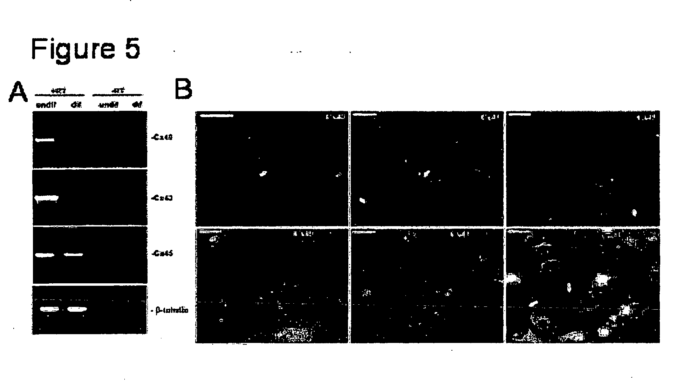

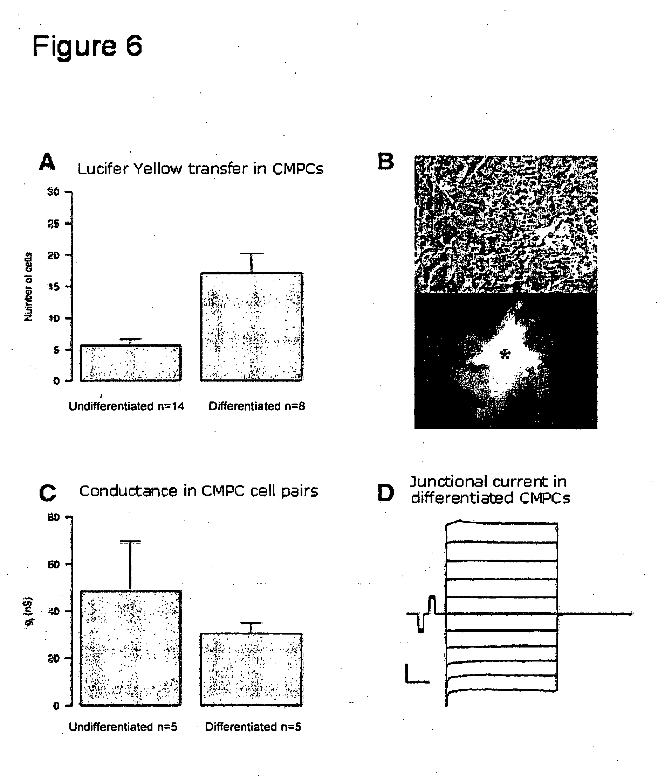

Cardiomyocytes, suitable for transplantation, should be able to couple correctly and propagate action potentials. Therefore, we analyzed the presence of gap junctional communication in hCMPCs. Semi-quantitative RT-PCR showed that undifferentiated hCMPCs express Cx40, Cx43 and Cx45. Connexin (Cx) are structural proteins needed to form gap junctions in the cell membrane. Upon differentiation into cardiomyocytes expression of Cx40 and 43 but not Cx45 is down regulated (Fig. 5a). Immunohistochemical analysis showed that while undifferentiated hCMPCs express Cx 40, 43 and 45 in a diffuse and predominantly intracellular pattern (Fig. 5b), hCMPCs-derived cardiomyocytes express Cx40 and 43 at the cell borders in a typical gap junctional pattern. Cx45 was found both intracellular and at the cell membrane. Metabolic coupling was measured by Lucifer Yellow injection in monolayers of undifferentiated and differentiated hCMPCs, resulting in spreading of dye to 6 ± 1 (n=14) and 17 ± 3 (n=8) cells, respectively (Fig. 6A and B). The difference in dye spreading was statistically significant (p<0.007).

Macroscopic gap junctional conductance in cell pairs of undifferentiated and differentiated CMPCs was 48 ± 21 nS (n=5) and 31 ± 4 nS (n=5), respectively (Fig. 6C). Differences in average conductance were not statistically significant. A representative recording of differentiated hCMPCs (Fig. 6D) shows a mild voltage dependent inactivation of gap junctional conductance starting at a Vⱼ of +30 mV indicative for Cx43 channels the predominantly expressed isoform. These results indicate gap junctional communication between hCMPC derived cardiomyocytes.

Analysis of hCMPC cardiomyocyte action potential shape showed a low maximal diastolic potential, as compared to values we measured in human embryonic stem cell-derived cardiomyocytes, human fetal ventricular cardiomyocytes and mouse P19 embryonic carcinoma cell-derived cardiomyocytes (-67 ± 8 mV compared to-48 ± 2, -38.5 ± 1.6 and -54 ± 2 mV, respectively) ^{19,34}. Furthermore, and possibly as a consequence, the maximal upstroke velocity is higher (39 ± 4 V/s compared to 7.0 ± 0.8, 8.9 ± 4.3 and 10.0 ± 1.0 V/s). APD₉₀ of hCMPCs is comparable with values measured in human embryonic stem cell-derived cardiomyocytes and human fetal ventricular cardiomyocytes (483 ± 51 ms compared to 436.4 ± 55.3 and 370.0 ± 45.8 ms). hCMPCs express high levels of gap junction proteins both in the differentiated and the undifferentiated state. In hCMPC cardiomyocytes, expression is almost exclusively found on the plasma membrane. The expression pattern reflected functional gap junction channels as confirmed by a robust metabolic and electrical coupling. Interestingly, hCMPC cardiomyocytes have a phenotype that is even more mature than cultured ventricular cardiomyocytes isolated from 16 week Without being hereby bound to any theory, we consistently and repeatedly find APD-90 values in the CMPCs as isolated with the methods described herein that are higher than week 16 myocytes as explained above. It thus appears that the cardiomyocytes derived from our CMPCs are more mature than the ones which can be found in a 16 week foetus. The differentiation potential of the CMPCs described herein is thus towards more mature cells.

In terms of future therapy, transplantation of poorly coupled skeletal myoblasts in human hearts in a clinical trial resulted in ventricular tachyarrhythmias in some patients¹⁴. Proper intercellular coupling with host heart cells will be necessary in order to preserve conduction characteristics and will be among the most important criteria for determining whether hCMPCs can be taken forward to clinical trials. The combination of high upstroke velocity, low maximal diastolic potential and efficient gap junctional communication suggests that hCMPC cardiomyocytes will sufficiently support impulse propagation when transplanted. The characterization of the hCMPCs in this study identified a distinct population of cardiac progenitor cells that can be used to study human cardiomyocyte differentiation, and serve as a suitable source for transplantable human cardiomyocytes.

Further described is a pharmaceutical composition comprising the cardiomyocytes which are obtainable by the methods of the invention by use of the CMPCs as defined herein. The pharmaceutical composition optionally comprises a pharmaceutically active carrier and/or diluent. Examples of suitable pharmaceutical carriers are well known in the art and include phosphate buffered saline solutions, water, sterile solutions etc. Compositions comprising such carriers can be formulated by well known conventional methods.

It is also envisaged to provide a pharmaceutical composition comprising the "tubular vascular structures" and/or "vascular sprouts" which are obtainable by use of the CMPCs in the respective differentiation methods as defined herein.

In a further aspect, the present invention relates to a pharmaceutical composition comprising a cell fraction according to claim 1. Further described is a kit comprising this pharmaceutical composition, wherein said kit and/or pharmaceutical composition further comprises instruction manuals which guide the skilled person to differentiate these CMPCs into cardiomyocytes and/or the tubular structures and/or the vascular sprouts , for example by use of the differentiation methods as described. To this end, this kit and/or pharmaceutical composition may further comprise the substances/chemicals and/or cell culture equipment (culture dishes; antibodies for quality staining, differentiation agents etc.) which are useful for the differentiation protocol and optionally, a solvent, diluent, buffer for stabilizing and/or storing the compounds. To this end, it is envisaged that the pharmaceutical composition comprising the CMPCs and/or the kit comprising this pharmaceutical composition further comprises the demethylating agents, antioxidant agents members of the TGF-β family; VEGF etc. as described herein in detail in the context of the differentiation methods.

It is also envisaged to keep/maintain/encapsulate the CMPCs comprised in the pharmaceutical composition and/or the kit described in the paragraph above in, for example, biodegradable grafts. Examples of the biocompatible/biodegradable material have been exemplified herein elsewhere. To this end, it is also envisaged to keep/maintain/encapsulate the CMPCs together with members of, for example the TGF-β family (when aiming to differentiate into cardiomyocytes) and/or VEGF (when aiming to differentiating into the "tubular structures" or "vascular sprouts") in the biocompatible/biodegradable material/graft in order to achieve the desired differentiation of the CMPCs at their envisaged destination (see the paragraph below). It is envisaged that the members of the TGF-β family and/or VEGF are contained per se within the biocompatible/biodegradable material/graft together with the CMPCs and/or to add recombinant cells which are able to express and secrete these TGF-β family members and/or VEGF, preferably in the vicinity of the CMPCs. The mentioned recombinant cells are preferably also CMPCS and/or cardiomyocytes comprising the desired member of the TGFβ-family and/or VEGF as an expressible nucleic acid, preferably under the control of a suitable promoter.

It will be understood that it is also possible to further encapsulate the TGFβ-member and/or VEGF for example into microcapsule structures. Microencapsulation can be used to slow the release of the TGFβ family member and/or VEGF into the body. This may decrease toxic side effects by preventing high initial concentrations in the subject. The release pattern is typically first-order in which the rate decreases exponentially with time until the drug source is exhausted. In this situation, a fixed amount of drug is inside the microcapsule. The concentration difference between the inside and the outside of the capsule decreases continually as the contained agent diffuses.

Consequently, it is envisaged that the last step of the differentiation of the CMPCs may occur by way of providing a pharmaceutical composition comprising the CMPCs-, preferably pre-differentiated with a demethylating agent and more preferred also in the presence of an antioxidant agent (these differentiation steps have been described herein before and the so-treated cells are denoted "pre-differentiated" in the following) and a member of the TGFβ-family (either directly or produced by a recombinant cell in the vicinity of the CMPC). Further described is a prodrug comprising pre-differentiated CMPCs. It is also envisaged to keep/maintain/encapsulate the CMPCs (preferably in the pre-differentiated form) in a first biodegradable/biocompatible graft and to add the TGFβ-member (either the compounds per se or cells producing them) in form of a second graft which is transplanted in the vicinity of the first graft and thereby to achieve the differentiation of the cells at their desired destination. Said second graft may be replaced if necessary or removed once the CMPCs have been differentiated into cardiomyocytes. The same applies equally to the tubular structures/vascular sprouts as mentioned herein before, by using the respective differentiation method described herein (including the treatment of the cells with an biologically active agent having the capabilities to induce angiogenesis or sprouting angiogenesis (like e.g. VEGF).

Further described is a pharmaceutical composition comprising the CMPCs together with members of the TGF-β family member in a biocompatible/biodegradable material/graft. Said pharmaceutical composition may alternatively comprise two distinct grafts, namely a first biodegradable/biocompatible graft comprising the CMPCs (preferably in the pre-differentiated form) and a second graft comprising the mentioned TGFβ-member (either the compounds pre se or cells producing these TGFβ members). It is also envisaged that the second graft is made of a non-biodegradable material, for example in case that a recombinant cell expressing and secreting one or more of the mentioned TGFβ-members is included instead of TGFβ member(s) per se. In such a case it is envisaged that the graft containing the TGFβ family member producing cells is removed once the CMPCs and/or the pre-differentiated CMPCs are differentiated into cardiomyocytes. It follows that such a graft may also be comprised of a non-biodegradable material as it is removed anyhow. Such a non-biodegradable graft, however, is preferably biocompatible, i.e. it has the quality of not having toxic or injurious effects on biological systems.

The above equally applies to pharmaceutical compositions comprising the CMPCs together with compounds like VEGF which have the capability to differentiate the CPMPs into the tubular structures or the vascular sprouts as described.

The pharmaceutical compositions can be administered to the subject at a suitable dose. The dosage regimen will be determined by the attending physician and clinical factors. The pharmaceutical compositions comprising the cardiomyocytes should be brought into the border zone surrounding the infarcted area via e.g. intramyocardial injection, or by engrafting the 3D structure onto the damaged myocardium.

The pharmaceutical compositions can thus be used in cardiomyocyte replacement therapy and/or for the treatment of myocardial infarction or for ameliorating the effects of myocardial infarction, i.e. the present invention relates to the use of the CMPCs obtainable by the method of the invention for the preparation of a pharmaceutical composition to be used in a cardiomyocyte replacement therapy and/or for the treatment of myocardial infarction or for ameliorating the effects of myocardial infarction. "Cardiomyocyte replacement therapy" in this regard refers to the improvement of cardiac contractile force. The goal of cardiomyocyte replacement therapy is to replace the lost cardiomyocytes in the ventricular wall by viable contractile cells. Due to the dead myocytes and the development of a scar, the heart has less power to pump. Improvement of its contractile function will help to increase the ejection fraction.

As mentioned before, it is also envisaged to keep/maintain/encapsulate the cardiomyocytes and/or CMPCs as explained above and/or the tubular structures or vascular sprouts in, for example, biodegradable grafts. Examples of the biocompatible/biodegradable material include collagen, gelatin, fibrin, albumin, hyaluronic acid, heparin, chondroitin sulfate, chitin, chitosan, alginic acid, pectin, agarose, hydroxyapatite, polypropylene, polyethylene, polydimethylsiloxane, polymer or copolymer of glycolic acid, lactic acid or amino acid, a mixture of two or more kinds of the biocompatible materials above, and the like. A particularly preferred biocompatible material is collagen. It is also preferred to mix collagen with other biocompatible material described in the above. Collagen may be any collagen and includes, for example, collagen soluble in acid, collagen solubilized by enzyme (for example, atelocollagen, etc.), collagen solubilized by alkali, collagen having modified amino acid side chains, bridged collagen, collagen produced by genetic engineering, or the like. Collagen having modified amino acid side chains includes, for example, succinyl or methyl collagen, or the like. Bridged collagen includes, for example, collagen treated with glutaraldehyde, hexamethylenediisocyanate or polyepoxy compound, or the like (Fragrance J., 1989-12, 104-109, Japanese Patent KOKOKU (examined) NoJ (1995)-59522)). The pharmaceutical compositions comprising the cardiomyocytes in such a biodegradable graft can then easily be brought into the border zone surrounding the infarcted area or onto the damaged myocardium.

It is further envisaged that the mentioned biocompatible/biodegradable grafts containing the cardiomyocytes and/or the CMPCs (preferably the pre-differentiated CMPCs) and/or the tubular structures or vascular sprouts and/or the member of the TGFβ-family or an biologically active agent having the capabilities to induce angiogenesis or sprouting angiogenesis (like e.g. VEGF) (depending on which differentiation result is to be achieved) are superimposed on a stent or stent graft. In the medical field, a stent is an expandable wire form or perforated tube (for example perforated by means of laser cutting) that is normally inserted into a natural conduit of the body to prevent or counteract a disease-induced localized flow constriction and/or to support weak points in arteries. An endovascular stent graft is a tubular device which is composed of fabric supported by another rigid structure, usually metal. This rigid structure is also called a stent.

It is also envisaged to superimpose the biocompatible/biodegradable grafts containing the cardiomyocytes and/or the CMPCs (preferably the pre-differentiated CMPCs) and/or the tubular structures or vascular sprouts as described and/or the member of the TGFβ-family or an biologically active agent having the capabilities to induce angiogenesis or sprouting angiogenesis (like e.g. VEGF) (depending on which differentiation result is to be achieved) on an artificial heart valve or a biological heart valve. An artificial heart valve is a device which is implanted in the heart of a subject, for example a human. These mechanical heart valves are prosthetics designed to replicate the function of the natural valves of the heart. Biological heart valves which are heart valves of animals (i.e. xenografts), like pigs, which undergo several chemical procedures in order to make them suitable for implantation in the human heart are also described herein.

Alternativley, transplanted heart valves of humans are envisaged (allograft or homograft).

As described, the biodegradable graft may be replaced by a biocompatible but non-degradable graft. Biocompatible means that it has the quality of not having toxic or injurious effects on biological systems, particular on the subject to be treated

It is further envisaged that the cardiomyocytes and/or the CMPCs (preferably the pre-differentiated CMPCs) and/or the tubular structures or vascular sprouts and/or the member of the TGFβ-family or an biologically active agent having the capabilities to induce angiogenesis or sprouting angiogenesis (like e.g. VEGF) (depending on which differentiation result is to be achieved) is(are) directly superimposed (which means without a biocompatible/biodegrabdable graft) on the above-mentioned devices, i.e. onto the stents, heart valves etc. Alternatively, the surface of the above mentioned devices can be coated with adhesive polymeric layers including but not limited to for example poly-L-Lysine (PLL) or fibronectin (FN).

The terms "treatment", "treating" and the like are used herein to generally mean obtaining a desired pharmacological and/or physiological effect. The effect may be therapeutic in terms of partially or completely curing a disease and/or adverse effect attributed to the disease. The term "treatment" as used herein covers any treatment of a disease in a mammal, particularly a human, and includes: (a) inhibiting the disease, i.e. arresting its development; or (b) relieving the disease, i.e. causing regression of the disease. Described is the treatment of patients with myocardial infarction. This treatment would involve inhibiting or relieving any medical condition related to myocardial infarction.

"Myocardial infarction" literally means that there is destruction of heart muscle cells due to a lack of oxygen. The resulting oxygen shortage causes damage and potential death of heart tissue.

The hCMPC-derived cardiomyocytes had a rather mature electrical phenotype in culture, and therefore will serve as a source of autologous transplantable cardiomyocytes.

As described, cardiomyocytes obtainable by the methods of the invention can be used for the preparation of a pharmaceutical composition, wherein said composition comprises or consists of cardiomyocytes which were differentiated from CMPCs as defined herein, and wherein said CMPCs were derived from the patient to be treated (i.e. autologous treatment). It will be understood that "autologous" refers to the cells that are reimplanted in the same individual as they come from (autografts). In contrast, cells transplanted from a different individual are referred to as allogeneic or as an allograft. Such allogeneic cells, their uses, and pharmaceutical compositions comprising them, are also within the scope of the present description.

In an further aspect, the use of the CMPCs for the preparation of a pharmaceutical composition for the treatment of myocardial infarction or for ameliorating the effects of myocardial infarction is described, wherein said "preparation" explicitly includes the differentiating methods of the present invention (i.e. the methods for the differentiation of the CMPCs of the invention to the cardiomyocytes as explained herein).

In a further aspect, the present description relates to a method for the treatment of myocardial infarction or for ameliorating the effects of myocardial infarction in a subject, comprising the methods of the invention as mentioned above (i.e. the methods for providing the cardiomyocytes as described) and further administering the so obtained cardiomyocytes to a subject in need thereof (e.g. the same subject from which the CMPCs were derived from (autograft) or to a different individual (allograft)).

Further described is a method for the treatment of myocardial infarction or for ameliorating the effects of myocardial infarction in a subject, comprising providing the CMPCs and further comprising the differentiating methods of the present invention (i.e. the methods for the differentiation of the CMPCs into the cardiomyocytes as explained herein) and further administering the so obtained cardiomyocytes to a subject in need thereof (e.g. the same subject from which the CMPCs were derived from (autograft) or to a different individual (allograft)).

In the context of the present description the term "subject" means an individual in need of a treatment of an affective disorder. Preferably, the subject is a mammalian, particularly preferred a human, a horse, a camel, a dog, a cat, a pig, a cow, a goat or a fowl.

The term "administered" means administration of a therapeutically effective dose of the cardiomyocytes as disclosed herein. By "therapeutically effective amount" is meant a dose that produces the effects for which it is administered. The exact dose will depend on the purpose of the treatment, and will be ascertainable by one skilled in the art using known techniques.

The hCMPC-derived cardiomyocytes had a rather mature electrical phenotype in culture, suggesting that they may serve as a homogeneous population of human cardiomyocytes for drug screens. For drug screens, one is in need of a homologues cell culture of cardiomyocytes which does not contain significant amount of other cells heterologous thereto as this might influence the behavior/response to the drug. Having a more mature phenotype of the cardiomyocytes will mimic more the patient situation, and a more mature cardiomyocyte, as the ones described herein, are more fragile and might respond different then more fetal cells. These cardiomyocytes are thus particularly useful for screening methods like drug screening methods.

It is envisaged that the CMPCs and thereby also the cardiomyocytes and/or "tubular structures" derived therefrom may comprise a foreign polynucleotide sequence which allows the transcription of nucleic acids and/or the expression of proteins encoded by such nucleic acids.

"Foreign" in this regard means that the polynucleotide sequence comes from the outside of the cell (i.e. is exogenous thereto) and is artificially introduced into the cells.

Preferably, the foreign polynucleotide sequences are part of a vector, e.g. a commercially available vector. Non-limiting examples include plasmid vectors compatible with mammalian cells, such as pUC, pBluescript (Stratagene), pET (Novagen), pREP (Invitrogen), pCRTopo (Invitrogen), pcDNA3 (Invitrogen), pCEP4 (Invitrogen), pMC1 neo (Stratagene), pXT1 (Stratagene), pSG5 (Stratagene), EBO-pSV2neo, pBPV-1 , pdBPVMMTneo, pRSVgpt, pRSVneo, pSV2-dhfr, pUCTag, pIZD35, pLXIN and pSIR (Clontech) and pIRES-EGFP (Clontech). For vector modification techniques, see Sambrook and Russell (2001), loc. cit. Vectors can contain one or more replication and inheritance systems for cloning or expression, one or more markers for selection in the host, e. g., antibiotic resistance, and one or more expression cassettes.

The coding sequences inserted in the vector can be synthesized by standard methods, isolated from natural sources, or prepared as hybrids. Ligation of the coding sequences to transcriptional regulatory elements (e. g., promoters, enhancers, and/or insulators) and/or to other amino acid encoding sequences can be carried out using established methods.

Furthermore, the vectors may comprise expression control elements, allowing proper expression of coding regions in suitable hosts. Such control elements are known to the skilled artisan and may include a promoter, translation initiation codon, translation and insertion site or internal ribosomal entry sites (IRES) (Owens, Proc. Natl. Acad. Sci. USA 98 (2001), 1471-1476) for introducing an insert into the vector. Preferably, the foreign nucleic acid molecule is operatively linked to said expression control sequences allowing expression in eukaryotic cells. Particularly preferred are in this context control sequences which allow for correct expression in cardiomyocytes and/or cells derived from the heart. Such control sequences have been exemplified herein elsewhere.

Control elements ensuring expression in eukaryotic cells are well known to those skilled in the art. As mentioned above, they usually comprise regulatory sequences ensuring initiation of transcription end optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in for example mammalian host cells comprise the CMV-HSV thymidine kinase promoter, SV40, RSV-promoter (Rous sarcome virus), human elongation factor 1α-promoter, CMV enhancer, CaM-kinase promoter or SV40-enhancer.

An expression vector as described is at least capable of directing the replication, and preferably the expression, of the nucleic acids and protein. Suitable origins of replication include, for example, the Col E1 , the SV40 viral and the M 13 origins of replication. Suitable promoters include, for example, the cytomegalovirus (CMV) promoter, the lacZ promoter, the gal10 promoter and the Autographa californica multiple nuclear polyhedrosis virus (AcMNPV) polyhedral promoter. Suitable termination sequences include, for example, the bovine growth hormone, SV40, LacZ and AcMNPV polyhedral polyadenylation signals. Examples of selectable markers include neomycin, ampicillin, and hygromycin resistance and the like. Specifically-designed vectors allow the shuttling of DNA between different host cells e.g. between bacteria-animal cells.

It is envisaged that the foreign polynucleotide sequence comprises a nucleic acid sequence (e.g. a gene, cDNA etc) which when expressed shows an effect on proliferation, hypertrophy, maximal diastolic potential, the ability to couple correctly and propagate action potentials; on excitation-contraction coupling and/or on cardiomyocyte and/or CMPC survival or death. It is furthermore envisaged that the foreign polynucleotide sequence comprises a gene which expresses a desired gene product which might have a beneficial effect on heart tissue and/or on angiogenesis. Genes which are envisaged in this regard are exemplified by but not limited to the following list: Anti apoptotic genes like Akt, pro angiogenesis like VEGF, pro growth like bFGF (but then driven by a cardiomyocyte specific promoter so it will not be on in undifferentiated cells so it will not block there differentiation. BMP2/4 which are pro myocyte differentiation.

It is also envisaged that the foreign polynucleotide sequence additionally or solely comprises a reporter gene, i.e. nucleic acid sequences encoding easily assayed proteins like for example GFP, EGFP, CAT, GAL or GUS. Reporter genes can be attached to other nucleic acid sequences so that only the reporter protein is made or so that the reporter protein is fused to another protein (fusion protein). Alternatively, only the reporter gene is expressed for example under the control of a cardiac promoter or under the control of a cardiomyocyte a or CPMC-specific promoter. Such promoters have been exemplified herein elsewhere. Suitable cardiac promoters are for example human cardiac actin promoter and the Mlc-2V promoter.

Alternatively, it is also envisaged that the cells described herein (i.e. the CMPCs, the cardiomyocytes and/or the "tubular structures" derived therefrom) may comprise inhibitory short nucleic acid molecules like anti-sense nucleotides, iRNA, siRNA, miRNA or ribozyme. These inhibitory molecules interfere with the transcription and/or expression of a target gene. miRNAs are short non-coding small regulatory RNAs that regulate protein expression by translational repression. In the past few years, studies have demonstrated that microRNAs (miRNAs) are important for the transcriptional regulation of target genes, serving important regulatory functions in angiogenesis, heart development, and maintenance of stem cell populations.

An siRNA approach is, for example, dislosed in Elbashir ((2001), Nature 411, 494-498)). It is also envisaged that for example short hairpin RNAs (shRNAs) are employed. The shRNA approach for gene silencing is well known in the art and may comprise the use of st (small temporal) RNAs; see, inter alia, Paddison (2002) Genes Dev. 16, 948-958. Approaches for gene silencing are known in the art and comprise "RNA"-approaches like RNAi or siRNA. Successful use of such approaches has been shown in Paddison (2002) loc. cit., Elbashir (2002) Methods 26, 199-213; Novina (2002) Mat. Med. June 3, 2002; Donze (2002) Nucl. Acids Res. 30, e46; Paul (2002) Nat. Biotech 20, 505-508; Lee (2002) Nat. Biotech. 20, 500-505; Miyagashi (2002) Nat. Biotech. 20, 497-500; Yu (2002) PNAS 99, 6047-6052 or Brummelkamp (2002), Science 296, 550-553. "Anti-sense" and "antisense nucleotides" means DNA or RNA constructs which block the expression of the naturally occurring gene product. As used herein, the terms "antisense oligonucleotide" and "antisense oligomer" are used interchangeably and refer to a sequence of nucleotide bases that allows the antisense oligomer to hybridize to a target sequence in an RNA by Watson Crick base pairing, to form an RNA: oligomer heteroduplex within the target sequence. The term "target sequence" in this regard refers to the sequence of the gene to be silenced within the cells. The antisense may have exact sequence complementarity to the target sequence or near complementarity. Such antisense oligomers may block or inhibit translation of the mRNA containing the target sequence, or inhibit gene transcription by binding to double-stranded or single stranded sequences. Preferably, said antisense oligonucleotides as used herein are "nuclease-resistant" oligomeric molecule e.g. their backbone is not susceptible to nuclease cleavage of a phosphodiester bond. Exemplary nuclease resistant antisense oligomers are oligonucleotide analogs, such as phosphorothioate and phosphate-amine DNA (pnDNA), both of which have a charged backbone, and methylphosphonate, morpholino, and peptide nucleic acid (PNA) oligonucleotides, all of which may have uncharged backbones.

Such cells as described above are well suited for, e.g., the screening methods and/or for pharmacological studies of drugs in connection with heart infarct and/or for the medical/pharmaceutical compositions/uses and/or treatments as described herein.

In a further aspect, the use of the cardiomyocytes and/or "tubular structures" and/or "vascular sprouts" for screening methods is described, preferably drug screening methods, said screening methods comprising the steps of:
(a) providing the cardiomyocytes and/or "tubular structures" and/or "vascular sprouts";
(b) bringing said cardiomyocytes and/or "tubular structures" and/or "vascular sprouts" into contact with a test substance; and
(c) evaluating the effect of said test substance on the phenotype of said cardiomyocytes and/or "tubular structures" and/or "vascular sprouts".

Evaluating the effect on the phenotype of the cardiomyocytes shall mean evaluating the effect on proliferation, hypertrophy, maximal diastolic potential, the ability to couple correctly and propagate action potentials; on excitation-contraction coupling, cardiomyocyte survival or death, and induction of genes known to be involved in these processes.

Evaluating the effect on the phenotype of the "tubular structures" and/or "vascular sprouts" shall mean evaluating the effect on the thickness of the sprouts, the length of the sprouts, the number of branching points and the presence of endothelial and smooth muscle cells.

The CMPCs can be employed for the screening methods as described. To this end, it is envisaged that (a) the CMPCs are provided and (b) said CMPCs are brought into contact with a test substance and (c) the effect of said test substance on the differentiation capability of the CMPCs is evaluated. "Differentiation capability" means the capability of the CMPCs to differentiate into either cardiomyocytes or "tubular structures" and/or "vascular sprouts", depending on the differentiation method (i.e. the differentiation methods). Said differentiation methods are explained in great detail herein.

The screening methods, optionally comprise the steps of a) introducing a molecular library of randomized candidate nucleic acids into a plurality of test-cells (i.e. the cardiomyocyetes and/or "tubular structures" and/or "vascular sprouts"), wherein each of said nucleic acids comprises a different nucleotide sequence; b) screening the plurality of test-cells for a cell exhibiting an altered phenotype. The methods may also include the steps of c) isolating the test-cell(s) exhibiting an altered phenotype; and d) isolating a candidate nucleic acid from the cell(s). The introduction (e.g. by way of retroviral vectors) and construction of suitable molecular libraries of randomized candidate nucleic acids is detailed, e.g. in WO 97/27213.

The term "test substance" in accordance with the screening methods of the present invention shall mean any biologically active substance that has an effect on proliferation, hypertrophy, cardiomyocyte survival or death, and induction of genes known to be involved in these processes. Screening of the effect on tubular structures are growth, ES and SMC survival, migration, sprout length, and branching points. Preferred compounds are nucleic acids, preferably coding for a peptide, polypeptide, antisense RNA, iRNA, siRNA, miRNA or a ribozyme or nucleic acids that act independently of their transcription respective their translation as for example an antisense RNA or ribozyme; natural or synthetic peptides, preferably with a relative molecular mass of about 1.000, especially of about 500, peptide analogs, polypeptides or compositions of polypeptides, proteins, protein complexes, fusion proteins, preferably antibodies, especially murine, human or humanized antibodies, single chain antibodies, Fab fragments or any other antigen binding portion or derivative of an antibody, including modifications of such molecules as for example glycosylation, acetylation, phosphorylation, farnesylation, hydroxylation, methylation or esterification, hormones, organic or inorganic molecules or compositions or libraries, preferably small molecules with a relative molecular mass of about 1.000, especially of about 500. The test-compounds can be recovered from products purified from natural sources or natural product mixtures, including bodily fluids, tissues and cells. These may be derived from bacteria, fungi, plants including higher plants, insects, and mammalians. It is particularly envisaged that the test- compound is a chemical and/or natural library of compounds, the latter including but not limited to libraries of natural compounds derived from flowers, green plants, fruits, vegetables, dairy products, grain and/or fungal sources.

It is envisaged that the term "contacting a cardiomyocyte and/or "tubular structure" and/or "vascular sprouts" with test substance" includes to introduce the test-compounds by suitable methods like electro/chemical poration; lipofection; bioballistics or microinjection into the cells.

It is also envisaged that the cells to be used in the screening assays of the present invention are used in a one-hybrid, three-hybrid or a one-two-hybrid-technique, which is a molecular biology technique used to discover protein-protein interactions or protein-DNA interactions by testing for physical interactions (such as binding) between two proteins or a single protein and a DNA molecule, respectively. Said terms and the biological principle behind are well known in the art.

Further described is a diagnostic composition comprising the cardiomyocytes and/or "tubular structures" and/or "vascular sprouts" and a kit comprising this diagnostic composition and instructions to use, i.e. instruction manuals which guide the skilled reader on how to keep the cells viable during the test protocol. To the more, it is envisaged that the above described kit and/or diagnostic compositions further comprise the test- substances (e.g. in the form of a library of compounds) and instructions to screen for the influence of said test substance(s) on the phenotype of the cells.

Further described is a diagnostic composition comprising the CMPCs as described herein and/or to a kit comprising this diagnostic composition, wherein said kit and/or diagnostic composition further comprises instruction manuals which guide the skilled person to differentiate these CMPCs into cardiomyocytes and/or "tubular structures" and/or "vascular sprouts", for example by use of the differentiation methods as described. To this end, this kit and/or diagnostic composition may further comprise the substances/chemicals and/or cell culture equipment (culture dishes; antibodies for quality staining etc) which are useful for the differentiation protocol and, optionally, a solvent, diluent, buffer for stabilizing and/or storing the compounds. To this end, it is envisaged that the diagnostic composition comprising the CMPCs and/or the kit comprising this diagnostic composition further comprises the demethylating agents, antioxidant agents members of the TGF-ß family etc as described herein in detail in the context of the differentiation methods of the invention. To the more, it is envisaged that the above described kit and/or diagnostic compositions further comprise the test-substances (e.g. in the form of a library of compounds) and instructions to screen for the influence of said test substance(s) on the phenotype of the cells.

This disclosure may best be understood in conjunction with the accompanying drawings, incorporated herein by references. Furthermore, a better understanding of the present invention and of its many advantages will be obtained from the following examples, given by way of illustration and are not intended as limiting.

The figures show
Figure 1: (A) Bright field images of CMPC cultures. Passage number is indicated. Right panel shows a MSC culture. B) Growth curve of freshly isolated CMPCs. Total number of cells per well were counted at the indicated day after plating. C) Table showing immunoreactivity of the indicated marker proteins by FACS analysis. The CMPC surface expression was compared to the levels in whole blood. (D) Semi quantitative RT-PCR on RNA isolated from undifferentiated CMPCs probed for the expression of the indicated genes. E) Immunolabeling against transcription factors GATA-4, Nkx2.5, Islet-1 and Western-Blot analysis to demonstrate the presence of GATA-4 protein.
Figure 2: (A) Semi-quantitative RT-PCR on RNA isolated from undifferentiated CMPCs (CMPC) and differentiated CMPCs (diff CMPC) probed for the expression of Islet-1, early cardiac transcription factors Nkx2.5 and GATA-4, the contractile proteins cardiac actin, cardiac Troponin I, MLC2v, and the skeletal myoblast transcription factor Myo-D. B) Immunolabeling of differentiated cell culture 3 weeks after 5-Azacytidine treatment against the contractile proteins α-actinin and cardiac Troponin I. C) Immunolabeling against the contractile proteins desmin, titin, MLC2v, β-MHC and α-actinin as expressed in CMPCs differentiated into cardiomyocytes. Right panels show the expression profile of the intercalated disk related proteins ZO-1 and N-cadherin. Bar = 20 µm.
Figure 3: (A) Western blot analysis on protein samples isolated from CMPCs stimulated without or with 1 ng/ml TGFβ or 25 ng/ml BMP-6 and probed with α-pSmad1 or α-pSmad2. Asterisk indicates an non-specific band showing equal loading. B) Semi-quantitative RT-PCR on RNA isolated from CMPCs stimulated without or with 1 ng/ml TGFβ for the indicated days probed for the expression of the early cardiac transcription factors Nkx2.5 and Mef2C, the contractile proteins cardiac actin, cardiac Troponin I, desmin, ANP and MLC2v. β-actin was used as a control for equal RNA input. C) Western blot analysis on protein samples from CMPCs stimulated without or with 1 ng/ml TGFβ for the indicated days, probed for Nkx2.5 or α-actinin. D) Immunohistochemical labelling for α-actinin shows the degree of differentiation into cardiomyocytes within the cultures without (left) or with (right) TGFβ stimulation. Counterstaining of nuclei was performed using DAPI. Bar = 50 µm.
Figure 4: (A) Current clamp recording from a CMPC cardiomyocyte showing a rapid phase 0 depolarization and mild diastolic depolarization. (B) Table summarizing CMPC action potential parameters. (C) Combined calcium-imaging and current clamp recording showing excitation-contraction coupling in a CMPC cardiomyocyte. Scale bars: horizontal 500 ms, vertical -50 mV.
Figure 5: (A) Semi-quantitative RT-PCR on RNA isolated from undifferentiated undif and differentiated (dif) CMPCs probed for the expression of Cx40, Cx43 and Cx45. β-tubulin was used as a control for equal RNA input. - RT is the control in which reverse transcriptase was omitted during the reaction. B) Immunolabeling against the connexin isoforms Cx40, Cx43 and Cx45 in undifferentiated CMPCs (top panels) and CMPCs differentiated into cardiomyocytes (bottom panels). Bar = 25 µm.
Figure 6. (A) Transfer of Lucifer Yellow from the injected cell to surrounding cells showing metabolic coupling. Dye spreading is significantly higher in differentiated CMPCs compared to undifferentiated cells. (B) Photomicrograph of a representative Lucifer Yellow injection in differentiated CMPCs, phase-contrast (upper panel) and Lucifer Yellow fluorescence (lower panel). Asterisk marks the injected cell. (C) Quantification of electrical conductance in undifferentiated and differentiated CMPC cell pairs. (D) Representative recording or gap junctional currents in a differentiated CMPC cell pair. Scale bars: horizontal 200 ms, vertical -200 pA.

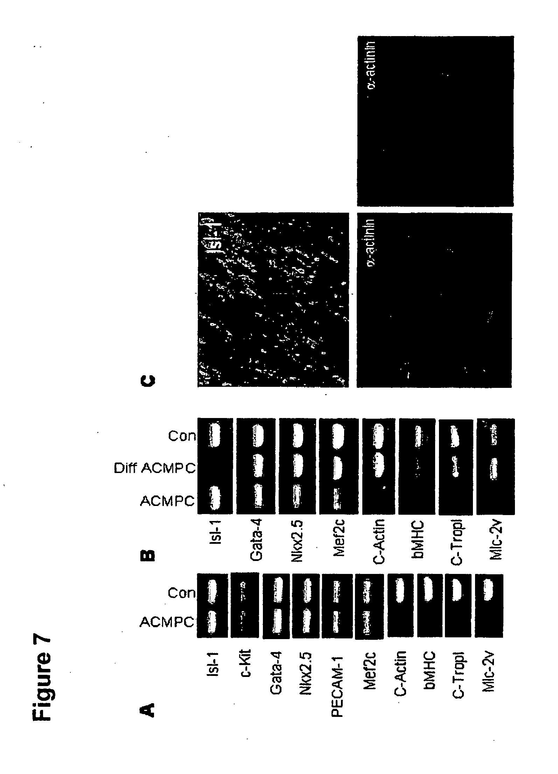

Figure 7. (A) Semi-quantitative RT-PCR on RNA isolated from undifferentiated adult (ACMPC) probed for the expression of the indicated genes. (B) Semi-quantitative RT-PCR on RNA isolated from undifferentiated adult (ACMPC) and ACMPC 4 weeks after differentiation, probed for the expression of the indicated genes. (C) Immunolabeling against Islet-1 in ACMPCs (top panel) and against α-actinin in ACMPCs differentiated into cardiomyocytes (bottom panels).

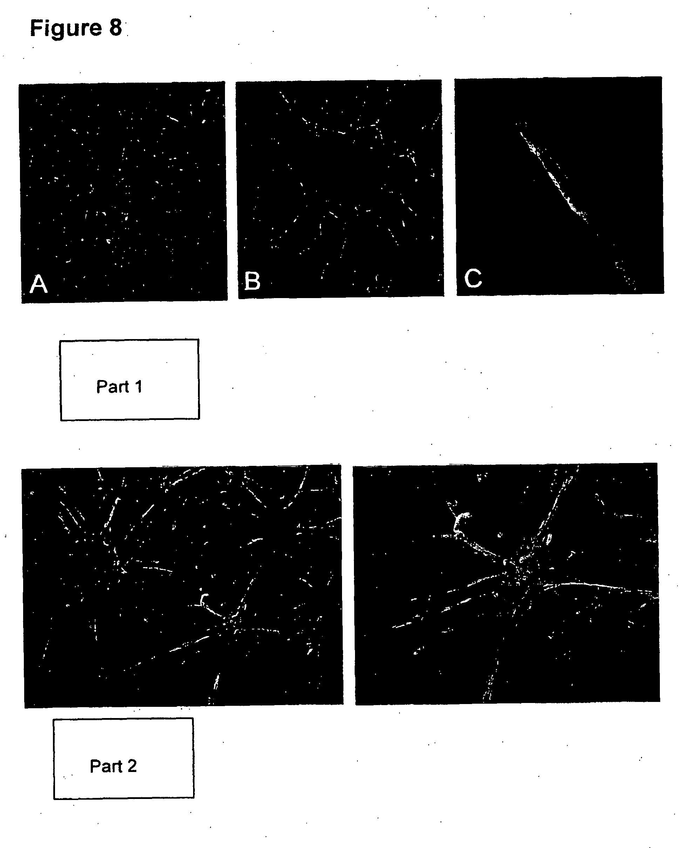

Figure 8: Results of an in vitro angiogenesis assay. Part 1: CMPC were plated on matrigel without (A) or with 100nM (B) miRNA-x. CMCPs formed tubularstructures with endothelial (red) and SMC-like (green) cells, as can be observed in the magnification (C). Adding miRNA-x resulted in less side-branches and increased tubular structures. Part 2: CMPC were plated on matrigel. CMCPs formed tubularstructures with endothelial (CD31 staining inred) and SMC-like (alpha-SMA in green) cells, as can be observed in the magnification.

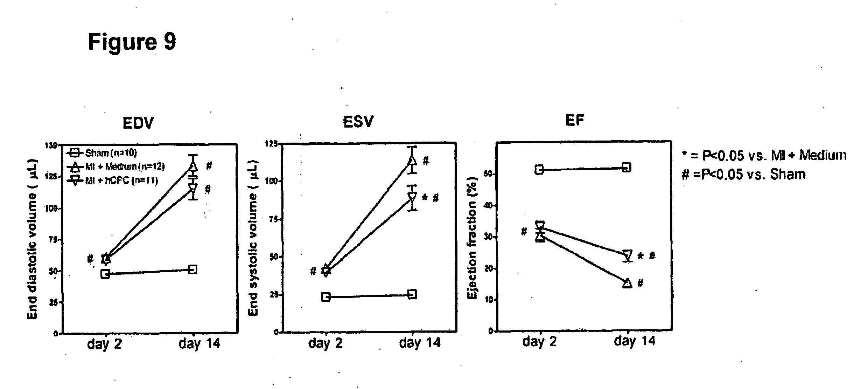

Figure 9: Results of a myocardial Infarction model in NOD/scid mice. At day 2" LV volumes were higher and EF (ejection fraction) was lower in both MI groups as compared to Sham, with no difference between the MI groups (Fig. 9). However at day 14, EF (ejection fraction) was higher and ESV (end systolic volume) was lower in the hCMPC group as compared to the MI+Medium group (P=0.001 and P=0.048 respectively). Although there was a trend towards a reduced EDV (end diastolic volume), this did not reach significance (P=0.14).

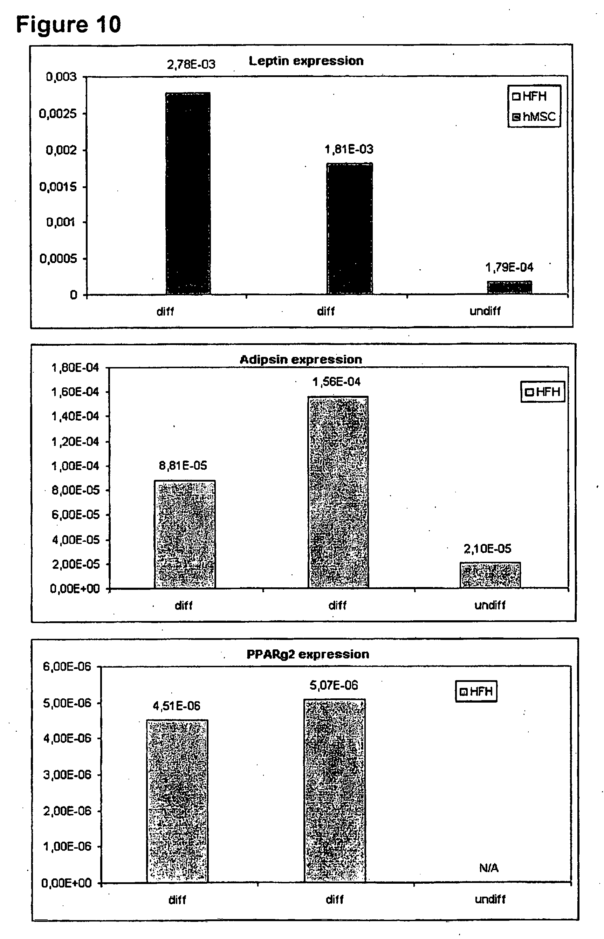

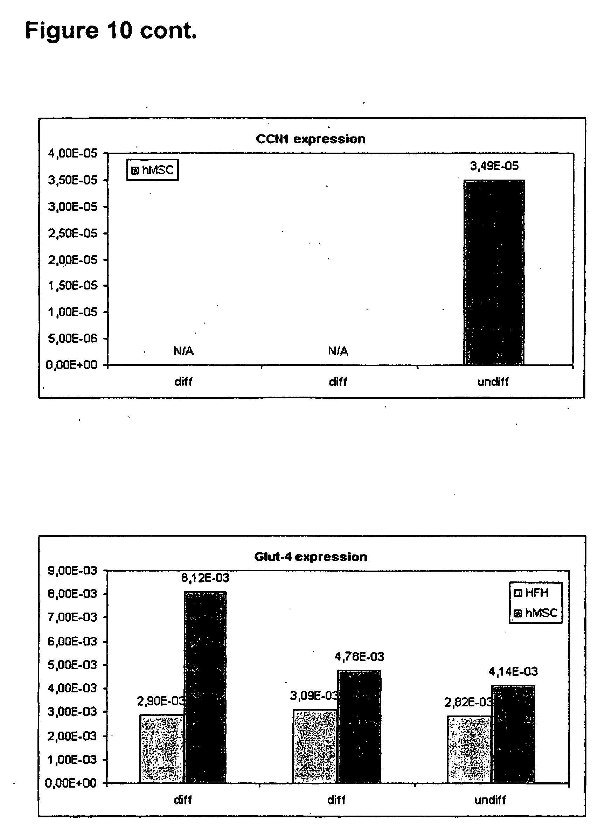

Figure 10: Results of adipocyte differentiation of the hCMPCs. Figures 10a to 10f show the evaluation of adipocyte differentiaton marker genes leptin, adisin, ppar, Cnn1/cyr61 and glut4.

### Examples

The following examples illustrate the invention. These examples should not be construed as to limit the scope of this invention. The examples are included for purposes of illustration and the present invention is limited only by the claims.

Group comparisons were made using one-way ANOVA with the Holm-Sidak posthoc test for multiple comparisons. Statistical significance was assumed if P < 0.05. All data are presented as mean ± SEM.

### Example 1: Isolation and culture of cardiomyocyte progenitor cells from human fetal hearts

For human fetal tissue collection and atrial biopsies, individual permission using standard informed consent procedures and prior approval of the ethics committee of the University Medical Center Utrecht were obtained. Fetal hearts were collected after elective abortion followed by Langendorff perfusion with Tyrode's solution, collagenase and protease. Atrial biopsies were minced into small pieces followed by collagenase treatment. After cardiomyocyte depletion of the cell suspension, cardiomyocyte progenitor cells were isolated by magnetic cell sorting (MACS, Miltenyl Biotec, Sunnyvale, CA) using Sca-1-coupled beads, following the manufactures protocol. Sca-1+ cells were eluted from the column by washing with PBS supplemented with 2% fetal calf serum (FCS) and cultured on 0.1% gelatin coated dishes in M199 (Gibco)/EGM (3:1) supplemented with 10% FCS (Gibco), 10 ng/ml basic Fibroblast growth factor (bFGF), 5 ng/ml epithelial growth factor (EGF), 5 ng/ml insuline like growth factor (IGF-1) and 5 ng/ml hepatocyte growth factor (HGF).

Clonal analysis was performed by limiting-dilution method. CMPCs were dissociated into single cells and plated into the wells of a gelatine-coated 96-well plate at a density of 0.5 cell per well in culture medium.

### Example 2: Differentiation of hCMPCs

To induce differentiation, cells were treated with 5 µM 5'-azacytidine (Sigma) for 72 hours in differentiation medium (Iscove's Modified Dulbecco's Medium /HamsF12 (1:1) (Gibco)) supplemented with L-Glutamine (Gibco), 2% horse serum, non-essential amino acids, Insulin-Transferrin-Selenium supplement , and 10⁻⁴ M Ascorbic Acid (Sigma)). After induction, the medium was changed every 3 days.

For TGFβ treatment, CMPCs were cultured in differentiation medium, stimulated with 5 µM 5'-azacytidine (Sigma) and 1 ng/ml TGFβ1 (Sigma) added 24 hours later. The medium was changed every 3 days.

### Example 3: RNA isolation and reverse transcription PCR

RNA was isolated using of Trizol (Invitrogen, Breda, The Netherlands) and reverse transcribed using oligo-dT Superscript 3 (Invitrogen). Primer sequences are given in table 1. The PCR reactions started with 2 min at 94 °C followed by 35 cycles of: 15 s at 94 °C, 30 s at 55 °C and 45 s at 72 °C. Products were analyzed on ethidium bromide-stained 1% agarose gel. β-tubulin or β-actin were used as RNA input control.

**Table1**

| **Gene** | **Primers** | **Size** |
|---|---|---|
| MEF2C | 5'-agatacccacaacacaccacgcgcc-3' | 192 |
| | 5'-atccttcacagagtcgcatgcgctt-3' | |
| Isl-1 | 5'-taagccaccgtcgtgtctc-3' | 107 |
| | 5'-tgatgaagcaactccagcag-3' | |
| GATA-4 | 5'-gacaatctggttaggggaagc-3' | 105 |
| | 5'-accagcagcagcgaggagat-3' | |
| NKX2.5 | 5'-cgccgctccagttcatag-3' | 111 |
| | 5'-ggtggagctggagaagacaga-3' | |
| MLC2V | 5'-gtcaatgaagccatccctgt-3' | 101 |
| | 5'-gcgccaactccaacgtgttct-3' | |
| TropT | 5'-gtgggaagaggcagactgag-3' | 131 |
| | 5'-atagatgctctgccacagc-3' | |
| bMyHC | 5'-gaagcccagcacatcaaaag-3' | 118 |
| | 5'-gatcaccaacaacccctacg-3' | |
| cActin | 5'-tcctgatgcgcatttttattc-3' | 123 |
| | 5'-aacaccactgctctagccacg-3' | |
| Desmin | 5'-acctgctcaacgtgaagatg-3' | 159 |
| | 5'-tggtatggacctcagaacc-3' | |
| MyoD | 5'-cggcggcggaactgctacgaa-3' | 458 |
| | 5'-ggggcgggggcggaaactt-3' | |
| c-kit | 5'-aagtggatggcacctgaaag-3' | 138 |
| | 5'-gaacttagaatcgaccggca-3' | |
| Cx40 | 5'-gagaagaagcagccagagtgtgaa-3' | 651 |
| | 5'-GACATGCAGGGTGGTCAGGAAGATT-3' | |
| Cx43 | 5'-agcgtgaggaaagtaccaaacagc-3' | 666 |
| | 5'-AAGAAGGCCACCTCAAAGATAGAC-3' | |
| Cx45 | 5'-ctagacccactgaaaagacc-3' | 494 |
| | 5'-TGATTTGCTACTGGCAGTGC-3' | |

### Example 4: Flow cytometric analysis

CMPCs were either used freshly isolated or as trypsinyzed cells after culture. 200.000 cells per sample was used for FACS analysis. The cells were washed twice in wash-buffer (wb: 1% FCS/PBS/0.05M azide) and re-suspended in 100 µl wb containing 0.5% antibody. Antibodies used for FACS analysis were FITC- or PE conjugated antibodies against CD31, CD34, CD105 (Endoglin), CD117 (c-kit), Sca1, and isotype control IgGs, all from Pharmingen BD.The cells were incubated on ice in the dark for 30 minutes, washed 4 times with cold wb, resuspended in 250 µl wb and analyzed using a Beckman Coulter Cytomics FC500 FACS.

### Example 5: Immunohistochemistry on cultured cells

Coverslips with cultured cells were rinsed in serum free medium, fixed in methanol (-20°C) or 4% paraformaldehyde, washed with PBS and permeabilized with 0.2% Triton X-100/PBS. Non-specific binding of antibodies was blocked with 2% bovine serum albumin (BSA). Incubation with primary antibodies was performed overnight in PBS/10% normal goat serum (NGS). Antibodies used recognized GATA-4 (Santa Cruz), Islets-1 (Hybridoma Bank), Cx40 (Chemicon), Cx43 (Zymed), Cx45 (kindly provided by Dr. T.H. Steinberg, Washington University, St.Louis, USA), α-actinin (Sigma), Troponin I (Chemicon) desmin (Sanbio), titin (Sigma), MLC2v (Alexis), β-MHC (kindly provided by Dr. A.F.M. Moorman, AMC Amsterdam, Netherlands), N-cadherin (Sigma) and ZO-1 (Zymed). Immunolabeling was performed using Texas Red (TR)- or fluorescein isothiocyanate (FITC)-conjugated secondary antibodies (Jackson Laboratories). All incubation steps were performed at room temperature and in between all incubation steps, cells were washed with PBS. Finally, coverslips were mounted in Vectashield (Vector Laboratories) and examined with a Nikon Optiphot-2 light microscope equipped for epifluorescence.

### Example 6: Western blot analysis

Western blot analysis was performed as described previously ³⁵. Detection was by ECL (Amersham). PSmad1 and PSmad2 rabbit polyclonal antibodies that specifically recognize phosphorylated Smad1/5 or Smad2 respectively were used 1:1000 (Cell signaling), Nkx2.5 rabbit polyclonal 1:500 (Santa Cruz), and α-actinin mouse monoclonal 1:5000 (Sigma). β-actin detection (1:10000, Chemicon) was used as a loading control.

### Example 7: Electrophysiology

A symmetrical setup with two HEKA EPC-7 patch clamp amplifiers was used to measure action potentials and electrical coupling between cells. Electrophysiology A symmetrical setup with two HEKA EPC-7 patch clamp amplifiers was used to measure action potentials and electrical coupling between cells. All signals were recorded using a custom data acquisition program (kindly provided by Dr. J.G. Zegers, AMC Amsterdam, The Netherlands) running on an Apple Macintosh computer equipped with a 12-bit National Instruments PCI-MIO-16E-4 acquisition card. Current signals were low-pass filtered at 2.5 kHz and acquired at 10 kHz. Calcium imaging combined with action potential recording was performed using a Cairn Research imaging system and an Axopatch 200B amplifier. All experiments were done using the whole cell patch clamp configuration. Action potential parameters measured from current clamp recordings were maximal diastolic potential, maximal upstroke velocity, action potential duration at 50 and 90 percent of repolarisation (APD50 and APD90).

Gap junctional macroscopic conductance in cell pairs was measured at a holding potential of -50 mV using a voltage clamp protocol that included 50 ms square pulses with an amplitude of +10 and -10 mV, followed by a 1 second square pulse ranging between -50 and +50 mV. Gap junctional conductance (gj) is defined as gj = Ij / Vj, where Ij and Vj denote junctional current and transjunctional voltage, respectively. By using the small prepulses for this calculation, gap junctional conductances were maximal and not inactivating. Offline analysis was done using MacDaq 8.0 (kindly provided by Dr. A.C.G. van Ginneken, AMC Amsterdam, The Netherlands) and R 2.0.1 (R Development Core Team, 2005).

Experiments were done at 20°C or, for calcium imaging, at 37°C. For calcium imaging, cells were loaded with 10 µM Fluo-3 AM for 25 minutes at 37°C. Extracullar buffer used in all cases was a modified Tyrode's solution, containing (in mmol/L) NaCl 140, KCl 5.4, CaCl2 1.8, MgCl2 1, HEPES 15, NaHCO3 35, glucose 6, pH 7.20/NaOH.

Action potentials were recorded using a pipette solution containing (in mmol/L) KCI 130, NaCl 10, HEPES 10, MgATP 5, MgCl2 0.5, pH 7.20/KOH. Pipette buffer used for conductance measurements contained (in mmol/L) potassium gluconate 125, KCl 10, HEPES 5, EGTA 5, MgCl2 2, CaCl2 0.6, Na2ATP 4, pH 7.20/KOH. For dye injection, microelectrodes were filled with 4% w/v Lucifer Yellow in 150 mM LiCl2, 10 mM HEPES and cells were injected for 2 minutes. Cells stained with Lucifer Yellow were counted, excluding the injected cell. Patch pipettes were pulled on a Narishige PC-10 puller and fire-polished. When filled with pipette buffer, the pipette resistance ranged between 2-5 MΩ. Liquid junction potentials were calculated using Clampex (Axon Instruments) and used for offline correction.

### Example 8: Microarray profiling of the genes expressed in the CMPCs

RNA was isolated from undifferentiated and differentiated CMPCs. 500ng of RNA and cRNA was made following standard illumina protocol (illumina RNA amplification kit, Ambion cat # I1755. We used illumina Sentrix® HumanRef-8 Expression BeadChip cat # BD-25-201 chips, and hybridization occurred at 55 degrees Celsius.

The genes listed in the following Table 3 are expressed in the CMCPs when measured with microarray gene profiling as indicated above.

**Table 3**

| Gene code | Accesion No | Gene code | Accesion No | Gene code | Accesion No |
|---|---|---|---|---|---|
| ZNF22 | NM_006963.1 | PTGDS | NM_000954.5 | THBS2 | NM_003247.2 |
| MYLK | NM_053031.1 | KIAA2028 | NM_172069.1 | COP | NM_052889.1 |
| ADH1B | NM_000668.3 | TNFRSF1B | NM_001066.2 | BDNF | NM_170734.2 |
| LOC255189 | XM_172929.3 | PODN | NM_153703.3 | | NM_015345.2 |
| PTGS1 | NM_080591.1 | LOC401151 | XM_379274.1 | THBS1 | NM_003246.2 |
| DDX3Y | NM_004660.2 | P8 | NM_012385.1 | AFAP | NM_021638.3 |
| FLJ23403 | NM_022068.1 | GAS6 | NM_000820.1 | LOC56901 | NM_020142.3 |
| INHBA | NM_002192.1 | ACTG2 | NM_001615.2 | MGP | NM_000900.1 |
| ADH1C | NM_000669.2 | CDKN2B | NM_004936.2 | NME4 | NM_005009.2 |
| C14orf141 | NM_032035.1 | CYP1B1 | NM_000104.2 | LOC375763 | XM_353489.1 |
| MAOA | NM_000240.2 | MGLL | NM_007283.4 | NFIC | NM_005597.2 |
| DHRS9 | NM_005771.3 | SRPUL | NM_014467.1 | C20orf97 | NM_021158.3 |
| COL8A1 | NM_020351.2 | LTBP2 | NM_000428.1 | ANGPTL4 | NM_016109.2 |
| EFEMP1 | NM_018894.1 | LOC400610 | XM_375478.1 | LOC51063 | NM_015916.3 |
| FLJ31166 | NM_153022.1 | CaMKIINalpha | NM_018584.4 | LOC375351 | XM_351558.1 |
| PLSCR4 | NM_020353.1 | PEG3 | NM_006210.1 | FLJ10948 | NM_018281.1 |
| ADH1A | NM_000667.2 | C20orf100 | NM_032883.1 | GDF5 | NM_000557.2 |
| KIAA1199 | NM_018689.1 | FLJ32389 | NM_144617.1 | ABCA8 | NM_007168.2 |
| CASP4 | NM_001225.2 | TNFAIP6 | NM_007115.2 | FBLN5 | NM_006329.2 |
| C7 | NM_000587.2 | MGC33637 | NM_152596.2 | DF | NM_001928.2 |
| CTSF | NM_003793.2 | MFAP4 | NM_002404.1 | CHI3L1 | NM_001276.1 |
| BF | NM_001710.3 | CTHRC1 | NM_138455.2 | C3 | NM_000064.1 |
| QSCN6 | NM_002826.2 | OLR1 | NM_002543.2 | C8orf13 | NM_053279.1 |
| PSMD5 | NM_005047.2 | CYBRD1 | NM_024843.2 | TRIM4 | NM_033091.1 |
| BTEB1 | NM_001206.1 | SPAG4 | NM_003116.1 | AMSH-LP | NM_020799.1 |
| SERPINA3 | NM_001085.2 | LRRC17 | NM_005824.1 | TNFRSF11B | NM_002546.2 |
| RUNX1 | NM_001754.2 | FBN1 | NM_000138.2 | LOC125476 | NM_194281.1 |
| UBE1 L | NM_003335.2 | ADAMTS5 | NM_007038.1 | TCF21 | NM_198392.1 |
| C1S | NM_001734.2 | STARD5 | NM_030574.2 | MAP3K5 | NM_005923.3 |
| | | A2M | NM_000014.3 | CDKN1A | NM_000389.2 |
| | | | | | |

| Gene code | Accesion No | Gene code | Accesion No | Gene code | Accesion No |
|---|---|---|---|---|---|
| FLJ20073 | NM_017654.2 | ICAM5 | NM_003259.2 | MGC17330 | NM_052880.2 |
| KIAA0779 | NM_015008.1 | TBX2 | NM_005994.3 | SPOCK | NM_004598.2 |
| PPARG | NM_005037.3 | CALD1 | NM_033138.2 | FLJ20254 | NM_017727.2 |
| LAMB2 | NM_002292.2 | IGFBP4 | NM_001552.1 | KLHL5 | NM_199039.1 |
| LOC375758 | XM_353487.1 | TM4SF1 | NM_014220.1 | C1orf24 | NM_052966.1 |
| SERPINF1 | NM_002615.3 | CEBPB | NM_005194.2 | 7h3 | NM_033025.4 |
| HNMT | NM_006895.1 | PP3856 | NM_145201.3 | MT2A | NM_005953.2 |
| LOC400718 | XM_375656.1 | EVC | NM_014556.2 | TCIRG1 | NM_006053.2 |
| FAM20C | NM_020223.1 | LOC375843 | XM_293449.2 | PLXND1 | NM_015103.1 |
| ALS2CR3 | NM_015049.1 | NR2F1 | NM_005654.3 | DKFZP566K1924 | NM_015463.1 |
| TRIM22 | NM_006074.2 | LTBR | NM_002342.1 | C1RL | NM_016546.1 |
| PSCD3 | NM_004227.3 | SELM | NM_080430.1 | TCF21 | NM_003206.2 |
| PPIL3 | NM_032472.3 | PLAU | NM_002658.1 | MXI1 | NM_130439.1 |
| TNIP2 | NM_024309.2 | MRF2 | XM_084482.3 | G1P3 | NM_002038.2 |
| LOC143903 | NM_178834.2 | TIMP1 | NM_003254.1 | B4GALT1 | NM_001497.2 |
| IFI16 | NM_005531.1 | PP1201 | NM_022152.3 | pp9099 | NM_025201.3 |
| ARC | NM_015193.2 | LTBP3 | NM_021070.2 | LOC401428 | XM_376715.1 |
| CPR8 | NM_020739.1 | CSRP1 | NM_004078.1 | CAV1 | NM_001753.3 |
| TNFRSF1A | NM_001065.2 | LOC285148 | XM_209490.3 | HLA-G | NM_002127.3 |
| RFX4 | NM_002920.3 | IFITM3 | NM_021034.1 | TNFRSF21 | NM_014452.3 |
| CKLFSF3 | NM_144601.2 | VIP32 | NM_021732.1 | AREG | NM_001657.2 |
| CDKN2A | NM_058196.1 | ASS | NM_000050.2 | VCAM1 | NM_080682.1 |
| NBL1 | NM_005380.3 | TORC3 | NM_022769.1 | PMP22 | NM_153322.1 |
| DDR2 | NM_006182.1 | BZRP | NM_007311.2 | PML | NM_033238.1 |
| RECK | NM_021111.1 | C1orf24 | NM_022083.1 | TACC1 | NM_006283.1 |
| LOC400810 | XM_375850.1 | SLC9A3R2 | NM_004785.1 | LOC388815 | XM_373927.1 |
| FSTL3 | NM_005860.1 | G0S2 | NM_015714.2 | PALM | NM_002579.1 |
| SIL1 | NM_022464.3 | RAB5C | NM_201434.1 | SPINLW1 | NM_020398.2 |
| COL16A1 | NM_001856.2 | KIAA1838 | NM_032448.1 | Spc25 | NM_020675.3 |
| S100A6 | NM_014624.2 | LGALS8 | NM_006499.3 | RCN3 | NM_020650.2 |
| | | | | | |

| Gene code | Accesion No | Gene code | Accesion No | Gene code | Accesion No |
|---|---|---|---|---|---|
| TNKS1BP1 | NM_033396.1 | KRTHB4 | NM_033045.2 | LRP1 | NM_002332.1 |
| TM4SF7 | NM_003271.3 | IGFBP7 | NM_001553.1 | KIAA2002 | XM_370878.1 |
| LOC154165 | XM_087859.4 | PTGIS | NM_000961.2 | H2AFJ | NM_177925.1 |
| TPRA40 | NM_016372.1 | ARHGEF6 | NM_004840.1 | ANGPTL2 | NM_012098.2 |
| HLA-C | NM_002117.3 | LOC253013 | XM_171892.1 | FHL1 | NM_001449.3 |
| AK3L1 | NM_016282.2 | FLJ23235 | NM_024943.1 | DFNA5 | NM_004403.1 |
| SEC14L1 | NM_003003.1 | TBX6 | NM_080758.1 | GABARAPL1 | NM_031412.1 |
| COPZ2 | NM_016429.1 | FLOT1 | NM_005803.2 | ARH | NM_015627.1 |
| C1QTNF5 | NM_015645.1 | SRGAP1 | NM_020762.1 | PDGFRL | NM_006207.1 |
| HLA-DMA | NM_006120.2 | RASD2 | NM_014310.3 | PCOLCE | NM_002593.2 |
| CTEN | NM_032865.3 | MAN2B1 | NM_000528.1 | XBP1 | NM_005080.2 |
| MGC15429 | NM_032750.1 | STAT1 | NM_139266.1 | SRPX | NM_006307.2 |
| EMP1 | NM_001423.1 | LOC201895 | NM_174921.1 | ACTN1 | NM_001102.2 |
| LOC388121 | XM_370864.1 | COL6A1 | NM_001848.1 | GYPC | NM_016815.2 |
| C20orf140 | NM_144628.1 | SCARF2 | NM_182895.1 | GNPTAG | NM_032520.3 |
| COL1A1 | NM_000088.2 | TFPI | NM_006287.3 | KIAA0233 | NM_014745.1 |
| STAT2 | NM_005419.2 | P4HA2 | NM_004199.1 | NEDL2 | XM_038999.2 |
| DPP4 | NM_001935.2 | FLJ21347 | NM_022827.2 | DKFZP586H2123 | NM_015430.1 |
| ACHE | NM_000665.2 | FBXO32 | NM_148177.1 | SLC7A5 | NM_003486.4 |
| LOC 124842 | XM_064333.4 | ANTXR1 | NM_032208.1 | ZNF226 | NM_015919.2 |
| ETFDH | NM_004453.1 | MMP23B | NM_006983.1 | LOC400741 | XM_378841.1 |
| LHX3 | NM_178138.2 | HIPK2 | NM_022740.1 | SARS | NM_006513.2 |
| PGCP | NM_016134.2 | FLJ36525 | NM_182774.1 | CAV2 | NM_198212.1 |
| RAB32 | NM_006834.2 | RAI14 | NM_015577.1 | STK17A | NM_004760.1 |
| KIAA0562 | NM_014704.1 | B2M | NM_004048.2 | HIST1 H2AH | NM_080596.1 |
| AXL | NM_001699.3 | CYB5R1 | NM_016243.1 | DCN | NM_133507.1 |
| LOC114990 | NM_138440.1 | MYH11 | NM_002474.1 | FOXF2 | NM_001452.1 |
| P5326 | NM_031450.2 | NEK6 | NM_014397.3 | PRO1914 | NM_014106.1 |
| LOC375531 | XM_353406.1 | SOD3 | NM_003102.1 | LOC375662 | XM_351780.1 |
| BACE2 | NM_138992.1 | MOCOS | NM_017947.1 | NDRG1 | NM_006096.2 |
| | | | | | |

| Gene code | Accesion No | Gene code | Accesion No | Gene code | Accesion No |
|---|---|---|---|---|---|
| FTL | NM_000146.2 | POU2F2 | NM_002698.1 | LOC375528 | XM_351672.1 |
| IFI35 | NM_005533.2 | TNFSF13 | NM_172089.1 | RPS4Y2 | NM_138963.1 |
| FLJ14800 | NM_032840.1 | KIAA1944 | XM_062545.5 | MKNK2 | NM_017572.2 |
| BRODL | NM_153252.2 | CCND1 | NM_053056.1 | FLJ10460 | NM_018097.1 |
| MED8 | NM_201542.1 | SAT | NM_002970.1 | TNN | XM_040527.5 |
| WDR10 | NM_052990.1 | PHTF2 | NM_020432.2 | LEMD2 | NM_181336.2 |
| RYR1 | NM_000540.1 | NOD9 | NM_170722.1 | FLJ22374 | NM_032222.1 |
| MTND5 | NM_173713.1 | SIAT7D | NM_175039.1 | PRRG1 | NM_000950.1 |
| SMARCD2 | NM_003077.2 | ARNT | NM_178426.1 | SEC22L2 | NM_012430.2 |
| SNTB2 | NM_130845.1 | AKR1C1 | NM_001353.4 | LOC376156 | XM_352094.1 |
| SAA4 | NM_006512.1 | TMSL1 | NM_182792.1 | CYGB | NM_134268.3 |
| RAP2B | NM_002886.2 | LOC138649 | XM_059987.6 | LOC374662 | XM_353096.1 |
| PPFIA4 | XM_046751.4 | STXBP1 | NM_003165.1 | LOC390690 | XM_372617.1 |
| LGALS12 | NM_033101.2 | MMP2 | NM_004530.1 | CABP2 | NM_031204.1 |
| KCNK2 | NM_014217.1 | FLJ14249 | NM_106552.1 | LOC400587 | XM_375426.1 |
| MGC40069 | NM_182615.1 | TIMP2 | NM_003255.2 | LOC400930 | XM_376019.1 |
| GAB2 | NM_080491.1 | TRIM44 | NM_017583.3 | LOC149345 | XM_086502.8 |
| S100A11 | NM_005620.1 | KIAA0545 | XM_032278.8 | TM4SF9 | NM_005723.2 |
| PLOD | NM_000302.2 | F8 | NM_019863.1 | PTPNS1 | NM_080792.1 |
| NNAT | NM_181689.1 | ANXA11 | NM_001157.2 | MGC13005 | NM_032685.2 |
| LMNA | NM_005572.2 | MGC32065 | NM_153271.1 | MICAL3 | XM_032997.4 |
| SCRN1 | NM_014766.2 | DKFZP564O0823 | NM_015393.1 | HIF1A | NM_181054.1 |
| SUMF1 | NM_182760.2 | KIAA1190 | NM_145166.2 | LMCD1 | NM_014583.2 |
| CD97 | NM_001784.2 | AEBP1 | NM_001129.2 | HMGCL | NM_000191.1 |
| ARHE | NM_005168.2 | KIAA0934 | NM_014974.1 | GLG1 | NM_012201.1 |
| LOC283111 | XM_210181.4 | LOC127545 | XM_060535.1 | FLJ90024 | NM_153342.1 |
| RNPEPL1 | NM_018226.2 | COL5A2 | NM_000393.2 | SULF2 | NM_198596.1 |
| HSPA12A | XM_048898.5 | HT008 | NM_018469.3 | LOC51334 | NM_016644.1 |
| LTC4S | NM_145867.1 | LOC286177 | XM_379582.1 | CREB5 | NM_004904.1 |
| SLC2A10 | NM_030777.3 | FLJ20920 | NM_025149.2 | MRVLDC1 | NM_031484.1 |
| | | | | | |

| Gene code | Accesion No | Gene code | Accesion No | Gene code | Accesion No |
|---|---|---|---|---|---|
| PDGFC | NM_016205.1 | RAFTLIN | NM_015150.1 | PRO0149 | NM_014117.2 |
| HS3ST3A1 | NM_006042.1 | SIGLEC8 | NM_014442.1 | ADRB2 | NM_000024.3 |
| CRAT | NM_144782.1 | SDC4 | NM_002999.2 | PTPNS1L2 | NM_178460.1 |
| ZSIG11 | NM_015926.3 | TAZ | NM_181314.1 | APG10L | NM_031482.3 |
| 01. Nov | NM_144663.1 | ARHC | NM_175744.3 | LOC126167 | XM_058997.5 |
| GRINL1A | NM_015532.2 | LOC285919 | XM_212094.1 | SLC1A5 | NM_005628.1 |
| KRTHA8 | NM_006771.3 | FLJ25224 | NM_182495.3 | SEMA4C | NM_017789.3 |
| LAMB1 | NM_002291.1 | LOC391403 | XM_372940.1 | FLJ23221 | NM_024579.1 |
| MAP3K6 | NM_145319.1 | DP1 | NM_005669.3 | BTBD6 | NM_033271.1 |
| SLC9A1 | NM_003047.2 | NINJ1 | NM_004148.2 | DIPA | NM_006848.1 |
| ABHD4 | NM_022060.1 | LOXL3 | NM_032603.2 | HSPB1 | NM_001540.2 |
| FLJ21977 | NM_032213.3 | SLB | XM_114272.2 | MGC44287 | NM_182607.2 |
| SPARC | NM_003118.1 | GRM4 | NM_000841.1 | KIFC3 | NM_005550.2 |
| EPB41 | NM_004437.1 | DKFZP564C103 | NM_015654.3 | DSCAML1 | NM_020693.2 |
| MVP | NM_005115.3 | ACTL6 | NM_016188.3 | U2AF1L3 | NM_144987.1 |
| DHX8 | NM_004941.1 | CGI-79 | NM_016024.1 | KIAA1944 | NM_133448.1 |
| CTDSP1 | NM_182642.1 | CCL14 | NM_032963.2 | AD-020 | NM_020141.2 |
| ARHGEF2 | NM_004723.2 | HAGHL | NM_032304.1 | CTSL | NM_145918.1 |
| LOC374402 | XM_353011.1 | CDC42EP1 | NM_007061.3 | SERPINB6 | NM_004568.4 |
| MGC3200 | NM_032305.1 | DSCR1 | NM_004414.5 | SLC9A8 | XM_030524.3 |
| TULP3 | NM_003324.3 | FLJ10647 | NM_018166.1 | NDST2 | NM_003635.2 |
| SLC35A1 | NM_006416.2 | RNF123 | NM_022064.2 | MFGE8 | NM_005928.1 |
| SF3B3 | NM_012426.2 | FLJ40113 | NM_198079.1 | PEA15 | NM_003768.2 |
| RDH5 | NM_002905.1 | CPA1 . | NM_001868.1 | LRP5 | NM_002335.1 |
| CISH | NM_145071.1 | TANK | NM_004180.2 | NMNAT1 | NM_022787.2 |
| FSTL1 | NM_007085.3 | C6orf29 | NM_032794.1 | ERO1L | NM_014584.1 |
| LOC375172 | XM_351416.1 | NAP1 L5 | NM_153757.1 | ABCC13 | NM_172024.1 |
| TMSL4 | NM_182794.1 | C7orf10 | NM_024728.1 | CBS | NM_000071.1 |
| BMP1 | NM_006129.2 | FXYD7 | NM_022006.1 | ACATE2 | NM_012332.1 |
| TUBG2 | NM_016437.1 | NARF | NM_031968.1 | FLJ32001 | NM_152609.1 |
| | | | | | |

| Gene code | Accesion No | Gene code | Accesion No | Gene code | Accesion No |
|---|---|---|---|---|---|
| QTRTD1 | NM_024638.2 | FLJ32675 | NM_173811.2 | BLVRB | NM_000713.1 |
| HECA | NM_016217.1 | GRLF1 | NM_004491.2 | KDR | NM_002253.1 |
| HCCA2 | NM_053005.2 | TAGLN | NM_003186.2 | PHLDA1 | NM_007350.1 |
| HIST1H2BK | NM_080593.1 | FLJ20522 | NM_017861.1 | CTSZ | NM_001336.2 |
| PLEKHC1 | NM_006832.1 | FLJ10916 | NM_018271.2 | RIN2 | NM_018993.2 |
| IL4R | NM_000418.1 | ZNF148 | NM_021964.1 | RPS3A | NM_001006.2 |
| KIAA1205 | XM_046305.3 | RASSF1 | NM_170712.1 | FBLN1 | NM_001996.2 |
| EXT1 | NM_000127.1 | HIST1 H2BD | NM_138720.1 | KIAA1600 | XM_049351.3 |
| RHOBTB3 | NM_014899.2 | LOC402176 | XM_377841.1 | SLC35B3 | NM_015948.2 |
| MAS1 | NM_002377.2 | MPG | NM_002434.1 | CDH11 | NM_001797.2 |
| DXS9928E | NM_004699.1 | ZNF33A | NM_006974.1 | ASC | NM_145183.1 |
| PILRA | NM_178273.1 | PKD1L2 | NM_182740.1 | RSU1 | NM_152724.2 |
| ATF5 | NM_012068.2 | GLS | NM_014905.2 | PTD015 | NM_024684.2 |
| DKFZp779M0652 | XM_374877.1 | SLC39A4 | NM_130849.1 | IER3 | NM_003897.2 |
| PLOD2 | NM_000935.1 | FLJ 13352 | NM_024592.1 | MGC29891 | NM_144618.1 |
| LOC377887 | XM_352835.1 | SE20-4 | NM_022117.1 | FREQ | NM_014286.2 |
| SLC22A2 | NM_003058.2 | BCL2L2 | NM_004050.2 | CLK3 | NM_001292.1 |
| NFKB1 | NM_003998.2 | RAD51L1 | NM_133510.1 | MGC10812 | NM_031425.1 |
| FLJ20542 | NM_017871.3 | CALCRL | NM_005795.2 | PJA2 | NM_014819.2 |
| MGC9850 | NM_152705.1 | FMOD | NM_002023.2 | RAB24 | NM_130781.1 |
| UBXD2 | NM_014607.2 | RABGGTA | NM_182836.1 | CREBL2 | NM_001310.2 |
| SH3YL1 | NM_015677.1 | GFOD1 | NM_018988.1 | LOC162427 | NM_178126.2 |
| SMOX | NM_175841.1 | SIAT6 | NM_174970.1 | ZNF339 | NM_021220.1 |
| KIAA1033 | XM_035313.6 | MT1J | NM_175622.2 | MGC14817 | NM_032338.2 |
| MDGA1 | NM_153487.2 | LOC255374 | XM_171171.2 | COL5A1 | NM_000093.2 |
| GADD45B | NM_015675.1 | BCL2L12 | NM_138639.1 | LOC401082 | XM_376257.1 |
| LZTS2 | NM_032429.1 | FIBCD1 | NM_032843.3 | SH3BGRL3 | NM_031286.2 |
| TNFRSF19 | NM_148957.2 | CALR | NM_004343.2 | ATP10D | NM_020453.2 |
| STEAP | NM_012449.2 | LZTR1 | NM_006767.1 | CD151 | NM_139030.2 |
| THBS3 | NM_007112.3 | SLC7A11 | NM_014331.2 | MGC33867 | NM_138346.1 |
| | | | | | |

| Gene code | Accesion No | Gene code | Accesion No | Gene code | Accesion No |
|---|---|---|---|---|---|
| FLJ21673 | NM_030898.2 | STX1A | NM_004603.1 | GNB5 | NM_006578.2 |
| NDEL1 | NM_030808.2 | ACTR1A | NM_005736.2 | RNF24 | NM_007219.2 |
| FLJ21174 | NM_024863.3 | EPN2 | NM_148921.2 | LOC125704 | XM_058931.6 |
| MGC15482 | NM_032875.1 | OPRS1 | NM_147158.1 | LIPT1 | NM_145198.1 |
| FLJ10375 | NM_018075.2 | HAP1 | NM_177977.1 | KIAA0759 | NM_015305.2 |
| CPSF2 | NM_017437.1 | DIAPH2 | NM_006729.2 | FAD104 | NM_022763.2 |
| DKFZP566E144 | NM_015523.1 | MAP4K1 | NM_007181.3 | ARPC5 | NM_005717.2 |
| FLJ23614 | NM_152695.2 | CD99L2 | NM_031462.1 | C6orf37 | NM_017633.1 |
| LOC51315 | NM_016618.1 | SLC25A28 | NM_031212.2 | FLJ23042 | NM_025157.2 |
| SHCBP1 | NM_024745.2 | TBL1X | NM_005647.2 | CNOT2 | NM_014515.1 |
| P24B | NM_007364.1 | ZHX1 | NM_007222.2 | LAIR1 | NM_021708.1 |
| TRIM11 | NM_145214.2 | SIAT1 | NM_003032.2 | USF2 | NM_003367.1 |
| ARD1 | NM_003491.2 | FLJ11273 | NM_018374.2 | HSGP25L2G | NM_017510.3 |
| LOC55831 | NM_018447.1 | KIAA0376 | XM_037759.5 | MSRB | NM_012228.2 |
| BNIP3L | NM_004331.1 | CCL21 | NM_002989.2 | OATL1 | XM_047025.6 |
| FLI1 | NM_002017.2 | CGI-128 | NM_016062.1 | ITCH | NM_031483.3 |
| DKFZP434P1750 | NM_015527.2 | PAX7 | NM_013945.1 | DXS1283E | XM_047871.6 |
| OAS1 | NM_002534.1 | APG7L | NM_006395.1 | HGS | NM_004712.3 |
| ADAM17 | NM_021832.1 | C20orf98 | NM_024958.1 | C20orf172 | NM_024918.2 |
| MUC3B | XM_168578.3 | SDSL | NM_138432.2 | CYLN2 | NM_032421.1 |
| DOCK11 | NM_144658.2 | PFKL | NM_002626.2 | PP1665 | NM_030792.4 |
| SCARB2 | NM_005506.2 | NUCB2 | NM_005013.1 | C10orf13 | NM_152429.2 |
| CDIPT | NM_006319.2 | RTN4 | NM_007008.1 | LOC92305 | NM_138385.1 |
| DEPDC2 | NM_025170.2 | IGF2R | NM_000876.1 | FBLP-1 | NM_017556.1 |
| KPNA6 | NM_012316.3 | FIBL-6 | NM_031935.1 | STK3 | NM_006281.1 |
| FAT | NM_005245.1 | PFAAP5 | NM_014887.1 | UBN1 | NM_016936.2 |
| FLJ12606 | NM_024804.1 | CDC10 | NM_001788.2 | LOC376138 | XM_352084.1 |
| SERPINE2 | NM_006216.2 | OGFR | NM_007346.2 | AGPAT1 | NM_032741.3 |
| TRIM8 | NM_030912.1 | DSTN | NM_006870.2 | SMAP-1 | NM_017979.1 |
| KIAA0769 | No_014824.1 | TTC17 | NM_018259.3 | B4GALT2 | NM_003780.2 |
| | | | | | |

| Gene code | Accesion No | Gene code | Accesion No | Gene code | Accesion No |
|---|---|---|---|---|---|
| UBXD1 | NM_025241.1 | STX5A | NM_003164.2 | IKBKB | NM_001556.1 |
| CDK5RAP2 | NM_018249.3 | LOC389562 | XM_374236.1 | GLCE | XM_290631.2 |
| TRIM47 | NM_033452.1 | STK4 | NM_006282.2 | CD63 | NM_001780.3 |
| KLK3 | NM_145864.1 | PPP1R12C | NM_017607.1 | FLJ34443 | NM_175918.2 |
| RAB3IL1 | NM_013401.2 | DHRS4 | NM_021004.2 | CSGlcA-T | XM_379974.1 |
| DHX34 | NM_014681.3 | PHC2 | NM_004427.2 | TTC13 | NM_024525.2 |
| KIAA1841 | XM_087056.4 | C6orf72 | NM_138785.1 | VAMP5 | NM_006634.2 |
| IPLA2(GAMMA) | XM_291241.3 | ANKRD13 | NM_033121.1 | RAC1 | NM_006908.3 |
| TNFRSF6B | NM_003823.2 | LOC339665 | XM_290973.1 | FLJ11280 | NM_018379.2 |
| FLJ10415 | NM_018089.1 | DDX24 | NM_020414.3 | SMARCD3 | NM_003078.2 |
| MGC19764 | XM_371039.2 | MGC955 | NM_024097.1 | FLJ32205 | NM_152561.1 |
| FARP1 | NM_005766.1 | C20orf194 | XM_045421.2 | GMPPA | NM_013335.2 |
| GPR1 | NM_005279.2 | SP140 | NM_007237.2 | FST | NM_013409.1 |
| FLJ25402 | NM_152567.1 | SIRPB1 | NM_006065.1 | ZNF16 | NM_006958.2 |
| LOC134478 | XM_068864.5 | APOBEC3A | NM_145699.2 | GCC1 | NM_024523.5 |
| SHMT2 | NM_005412.3 | CST3 | NM_000099.2 | GNA11 | NM_002067.1 |
| TNFSF15 | NM_005118.2 | OSBPL8 | NM_020841.3 | KIAA0256 | NM_014701.1 |
| TOM1 | NM_005488.1 | TMSL2 | NM_182793.1 | MAP3K3 | NM_002401.3 |
| ZCWCC2 | NM_024657.2 | VPS16 | NM_080414.1 | MKLN1 | NM_013255.2 |
| SELB | NM_021937.2 | PKD2 | NM_000297.2 | PPP5C | NM_006247.2 |
| MGC2749 | NM_024069.2 | LOC391023 | XM_372773.1 | GSN | NM_198252.1 |
| SLC39A3 | NM_144564.3 | RGL1 | NM_015149.2 | RPL39 | NM_001000.2 |
| C20orf149 | NM_024299.2 | SART2 | NM_013352.1 | MGC32020 | NM_152266.1 |
| SLC17A5 | NM_012434.3 | SLC35A4 | NM_080670.2 | LOC389359 | XM_374156.1 |
| ALG3 | NM_005787.3 | EPS15L1 | NM_021235.1 | BLZF1 | NM_003666.2 |
| HNRPDL | NM_005463.2 | ZNF554 | NM_152303.1 | SRPR | NM_003139.2 |
| JM5 | NM_007075.1 | MYO1B | NM_012223.2 | FLJ 12221 | XM_031342.1 |
| SLC43A3 | NM_017611.2 | FLNB | NM_001457.1 | LOC389445 | XM_371857.2 |
| KIAA0543 | XM_379967.1 | PRICKLE2 | NM_198859.1 | CNN2 | NM_004368.2 |
| DPYSL2 | NM_001386.3 | AKAP2 | NM_147150.1 | CGI-30 | NM_015958.1 |
| | | | | | |

| Gene code | Accesion No | Gene code | Accesion No | Gene code | Accesion No |
|---|---|---|---|---|---|
| MGC4549 | NM_032377.2 | MGC19604 | NM_080665.3 | CTNS | NM_004937.1 |
| GSTT1 | NM_000853.1 | TP53AP1 | NM_007233.1 | C5orf4 | NM_016348.1 |
| PHLDA2 | NM_003311.2 | SIAT4A | NM_173344.1 | MPZL1 | NM_003953.3 |
| LOC400320 | XM_375163.1 | CPSF1 | NM_013291.1 | DKFZP434J154 | NM_016003.1 |
| EPB49 | NM_001978.1 | CBLN1 | NM_004352.1 | PTD008 | NM_016145.1 |
| FHL2 | NM_001450.3 | MBTD1 | NM_017643.1 | CALM2 | NM_001743.3 |
| CBX6 | NM_014292.2 | SARA1 | NM_020150.3 | FLJ22679 | NM_032227.1 |
| PELO | NM_015946.4 | SIN3B | XM_050561.7 | KRTCAP2 | NM_173852.2 |
| MGC50836 | XM_171060.4 | CALU | NM_001219.2 | LOC399768 | XM_378228.1 |
| OCSP | NM_031945.2 | LOC92912 | NM_173469.1 | C14orf24 | NM_173607.2 |
| KIAA0802 | XM_031357.5 | BIRC2 | NM_001166.3 | FLJ46603 | NM_198530.1 |
| MPPE1 | NM_138608.1 | IMPA2 | NM_014214.1 | DUSP6 | NM_022652.2 |
| LOC339352 | XM_294910.1 | MIPEP | NM_005932.1 | FLJ31295 | NM_152320.1 |
| BMPR2 | NM_033346.2 | FBN2 | NM_001999.2 | FLJ00060 | NM_033206.1 |
| CYP4F3 | NM_000896.1 | PKD1-like | NM_024874.3 | RAI | NM_006663.1 |
| WSB2 | NM_018639.3 | LOC389816 | XM_372161.1 | ZDHHC18 | NM_032283.1 |
| CHST1 | NM_003654.2 | LOC374723 | XM_351066.1 | TMSL6 | NM_181428.1 |
| PLSCR3 | NM_020360.2 | KIAA0121 | XM_052386.3 | APG12L | NM_004707.2 |
| TM4SF10 | NM_031442.2 | C15orf17 | NM_020447.2 | ATP6V1F | NM_004231.2 |
| FXYD5 | NM_014164.3 | CTNND1 | NM_001331.1 | LOC402694 | XM_380042.1 |
| BBS1 | NM_024649.4 | FLJ22329 | NM_024656.2 | ZFYVE21 | NM_024071.2 |
| NDUFS7 | NM_024407.3 | MYL5 | NM_002477.1 | KIAA1223 | XM_048747.8 |
| EIF4EBP1 | NM_004095.2 | GMPPB | NM_021971.1 | AHR | NM_001621.2 |
| MGST1 | NM_145792.1 | IKBKG | NM_003639.2 | FLJ14360 | NM_032775.2 |
| KIAA0103 | NM_014673.2 | LOC375378 | XM_353337.1 | IL18 | NM_001562.2 |
| C20orf30 | NM_014145.3 | SMBP | NM_020123.2 | PRIC285 | NM_033405.2 |
| GPR10 | NM_004248.1 | CDKN1B | NM_004064.2 | SH2D3C | NM_170600.1 |
| SCAM-1 | NM_005775.2 | LOC375406 | XM_351591.1 | ACO1 | NM_002197.1 |
| EIF3S10 | NM_003750.1 | LOC253982 | NM_181718.3 | ZNF323 | NM_145909.1 |
| C14orf123 | NM_014169.2 | LOC390066 | XM_372359.1 | RAP1A | NM_002884.1 |
| | | | | | |

| Gene code | Accesion No | Gene code | Accesion No | Gene code | Accesion No |
|---|---|---|---|---|---|
| LOC389789 | XM_372140.2 | PCBP3 | NM_020528.1 | ZNF145 | NM_006006.3 |
| AGA | NM_000027.2 | COMT | NM_007310.1 | ECM1 | NM_022664.1 |
| DKFZp761D221 | NM_032291.1 | FAP | NM_004460.2 | SSX5 | NM_175723.1 |
| H2AFJ | NM_177925.1 | EMILIN1 | NM_007046.1 | CD44 | NM_000610.2 |
| SQRDL | NM_021199.1 | IFITM2 | NM_006435.1 | NS3TP2 | NM_023927.1 |
| MOXD1 | NM_015529.1 | ANGPT1 | NM_001146.3 | RPS4Y | NM_001008.2 |
| FLJ30681 | NM_133459.1 | BRIP1 | NM_032043.1 | TIMP3 | NM_000362.3 |
| ZNF345 | NM_003419.2 | SYTL2 | NM_032379.2 | TRADD | NM_003789.2 |
| CKTSF1B1 | NM_013372.4 | DOC1 | NM_014890.1 | ACAS2L | NM_032501.2 |
| GDF15 | NM_004864.1 | SPON2 | NM_012445.1 | C1QTNF1 | NM_198593.1 |
| ADFP | NM_001122.2 | TNXB | NM_032470.2 | EIF1AY | NM_004681.2 |
| COL1A2 | NM_000089.2 | TXNIP | NM_006472.1 | LOC400693 | XM_375609.1 |
| MAP1LC3A | NM_181509.1 | SLITRK6 | NM_032229.2 | DAB2 | NM_001343.1 |
| RARRES2 | NM_002889.2 | LOC374822 | XM_353149.1 | HCA112 | NM_018487.2 |
| LAMA4 | NM_002290.2 | AKR1C4 | NM_001818.2 | SEDL | NM_014563.2 |
| ZNF25 | NM_145011.2 | HLA-B | NM_005514.4 | MMP14 | NM_004995.2 |
| MGC3207 | NM_032285.1 | CTGF | NM_001901.1 | TWIST2 | NM_057179.1 |
| C21orf6 | NM_016940.1 | FLJ21986 | NM_024913.3 | LOC400682 | XM_375590.1 |
| INMT | NM_006774.3 | PRSS11 | NM_002775.2 | INHBE | NM_031479.3 |
| OXTR | NM_000916.3 | FLJ22625 | NM_024715.2 | MGC4504 | NM_024111.2 |
| LY96 | NM_015364.2 | STOM | NM_198194.1 | ATP8B4 | XM_370863.1 |
| KRTAP10-10 | NM_181688.1 | EIF5A2 | NM_020390.5 | ZHX3 | NM_015035.2 |
| LOC255849 | XM_172855.1 | KCNS1 | NM_002251.3 | LOC387758 | NM_203371.1 |
| NNMT | NM_006169.1 | SERF1A | NM_021967.1 | GDF10 | NM_004962.2 |
| NFIB | NM_005596.1 | KIAA1237 | XM_087386.5 | FLJ14525 | NM_032800.1 |
| AKR1C2 | NM_001354.4 | AKR1C3 | NM_003739.4 | CLDN11 | NM_005602.4 |
| IL1R1 | NM_000877.2 | LOC400738 | XM_378837.1 | FLJ20701 | NM_017933.3 |
| LOC399959 | XM_378316.1 | LOC374987 | XM_351264.1 | RAB13 | NM_002870.2 |
| C10orf10 | NM_007021.1 | MAGP2 | NM_003480.1 | KIAA0779 | XM_098229.9 |
| PTX3 | NM_002852.2 | ST6GALNAC6 | NM_013443.3 | LOC286343 | XM_210019.1 |
| FLJ32332 | NM_144641.1 | PPFIBP1 | NM_003622.2 | SLCO1A2 | NM_005075.2 |
| FLJ38149 | XM_375563.1 | LOC388019 | XM_373611.1 | GLMN | NM_053274.1 |
| DDIT3 | NM_004083.3 | PSMB8 | NM_148919.2 | UGCG | NM_003358.1 |
| PAPPA | NM_002581.3 | FLJ11286 | NM_018381.1 | SULT2B1 | NM_177973.1 |
| LOXL4 | NM_032211.5 | RASL12 | NM_016563.2 | NPDC1 | NM_015392.2 |
| CEBPD | NM_005195.2 | TREML1 | NM_178174.2 | ABTB1 | NM_172028.1 |
| LOC93349 | NM_138402.2 | PI16 | NM_153370.1 | LOC390046 | XM_372351.2 |
| LOC401217 | XM_376443.1 | DKFZp686O1689 | NM_182608.2 | MRPL4 | NM_146387.1 |
| BCL6 | NM_001706.2 | FCGRT | NM_004107.3 | STXBP5 | NM_139244.2 |
| ARF4L | NM_001661.2 | ANKRD25 | NM_015493.3 | LOC400768 | XM_378883.1 |
| TGFB1I1 | NM_015927.3 | K6HF | NM_004693.1 | LOC400649 | XM_378746.1 |
| PCDH18 | NM_019035.2 | JDP2 | NM_130469.2 | M17S2 | NM_031862.1 |
| COL6A2 | NM_001849.2 | EHD2 | NM_014601.2 | CDC42EP5 | NM_145057.2 |
| C9orf59 | NM_033387.2 | SPUVE | NM_007173.3 | CD59 | NM_000611.4 |
| DSIPI | NM_004089.2 | B7 | NM_006992.2 | PROS1 | NM_000313.1 |
| PDGFRB | NM_002609.2 | C9orf88 | NM_022833.1 | APM2 | NM_006829.1 |
| MICB | NM_005931.2 | EPS8 | NM_004447.3 | MBNL1 | NM_021038.2 |
| HPCAL1 | NM_002149.2 | LTBP4 | NM_003573.1 | LOC378157 | XM_353658.1 |
| EPLIN | NM_016357.1 | LRP10 | NM_014045.2 | CORO1B | NM_020441.1 |
| PLTP | NM_182676.1 | MGC15737 | NM_032926.2 | LOC255783 | NM_178511.2 |
| COL6A3 | NM_057167.1 | RGC32 | NM_014059.1 | WARS | NM_004184.2 |
| RRAS | NM_006270.2 | HOXA5 | NM_019102.2 | CXCL16 | NM_022059.1 |
| SERPING1 | NM_000062.1 | PRNP | NM_183079.1 | SPG20 | NM_015087.3 |
| TAP1 | NM_000593.4 | PDLIM2 | NM_198042.2 | RIS1 | NM_015444.1 |
| MGC16121 | NM_032762.2 | RNASE4 | NM_194431.1 | TRAM2 | NM_012288.1 |
| NCOA7 | NM_181782.2 | MGC10500 | NM_031477.2 | LCAT | NM_000229.1 |
| MGC3047 | NM_032348.2 | UACA | NM_018003.1 | CPNE7 | NM_153636.1 |
| DKFZp761G0122 | NM_152661.1 | PPP1 R3C | NM_005398.3 | NEXN | NM_144573.1 |
| PDLIM7 | NM_005451.3 | ARRDC4 | NM_183376.1 | OPTN | NM_021980.3 |
| FLJ11196 | NM_018357.2 | ZYX | NM_003461.3 | KCNRG | NM_199464.1 |
| | | | | | |

| Gene code | Accesion No | Gene code | Accesion No | Gene code | Accesion No |
|---|---|---|---|---|---|
| URG4 | NM_017920.2 | MTHFD2 | NM_006636.2 | FOXP1 | NM_032682.3 |
| STAT1 | NM_007315.2 | IQGAPI | NM_003870.2 | C1QTNF6 | NM_182486.1 |
| CNTNAP1 | NM_003632.1 | MGC50372 | NM_173566.1 | MGC23284 | XM_378628.1 |
| CBX3 | NM_007276.3 | ACTA2 | NM_001613.1 | C10orf6 | NM_144592.1 |
| INPP1 | NM_002194.2 | SLC12A4 | NM_005072.3 | ACSL4 | NM_004458.1 |
| FLJ37940 | NM_178534.2 | BCL3 | NM_005178.2 | FLJ11259 | NM_018370.1 |
| ZNF498 | NM_145115.1 | KIAA1949 | XM_166376.1 | GARS | NM_002047.1 |
| FLJ12409 | NM_025105.1 | LOC375460 | XM_351626.1 | CLIPR-59 | NM_015526.1 |
| LOC389414 | XM_374176.1 | LOC400653 | XM_378750.1 | GRK5 | NM_005308.1 |
| IL10RB | NM_000628.3 | GNB3 | NM_002075.2 | C11orf15 | NM_020644.1 |
| EVI5 | NM_005665.2 | MICA | NM_000247.1 | FLJ25348 | NM_144569.2 |
| LOX | NM_002317.3 | VEGFB | NM_003377.3 | GPR124 | NM_032777.6 |
| NXPH4 | NM_007224.1 | SMARCA2 | NM_139045.1 | CPD | NM_001304.3 |
| MGMT | NM_002412.1 | NTE | NM_006702.2 | BHD | NM_144997.3 |
| DDAH1 | NM_012137.2 | LOC155340 | XM_055725.3 | PLK3 | NM_004073.2 |
| THRA | NM_003250.4 | KRT15 | NM_002275.2 | FBLN2 | NM_001998.1 |
| TNC | NM_002160.1 | FLJ37396 | NM_173671.1 | CTSD | NM_001909.3 |
| SCGF | NM_002975.2 | TPSB2 | NM_024164.2 | RNH | NM_002939.3 |
| MGC14276 | NM_153248.2 | SBBI54 | NM_138334.1 | ANTXR2 | NM_058172.1 |
| NRXN3 | NM_138970.2 | LPPR2 | NM_022737.1 | MED8 | NM_052877.2 |
| CYP1A2 | NM_000761.2 | IL1R2 | NM_004633.3 | PCK2 | NM_004563.1 |
| BCR | NM_021574.1 | ADM | NM_001124.1 | LOC343069 | XM_291395.1 |
| FTH1 | NM_002032.1 | HCCR1 | NM_015416.2 | CD1C | NM_001765.1 |
| LOC146784 | XM_378694.1 | JM4 | NM_007213.1 | EFEMP2 | NM_016938.1 |
| KIAA1055 | NM_015079.2 | LAMC1 | NM_002293.2 | PGRMC2 | NM_006320.1 |
| PRKCDBP | NM_145040.1 | FER1L3 | NM_133337.1 | PPGB | NM_000308.1 |
| MACF1 | NM_012090.3 | OS-9 | NM_006812.1 | MMP17 | NM_016155.2 |
| DACH | NM_080759.1 | ITGB1 | NM_002211.2 | MME | NM_007288.1 |
| TM7SF1 | NM_003272.1 | LENG8 | NM_052925.1 | HTATIP2 | NM_006410.3 |
| C20orf18 | NM_031227.1 | FLJ35976 | NM_173639.1 | TAGLN2 | NM_003564.1 |
| | | | | | |

| Gene code | Accesion No | Gene code | Accesion No | Gene code | Accesion No |
|---|---|---|---|---|---|
| MOBKL2A | NM_130807.2 | LOC90271 | XM_030445.6 | PLEC1 | NM_000445.1 |
| ACSL3 | NM_004457.3 | USH1G | NM_173477.2 | ALDH1B1 | NM_000692.3 |
| A2LP | NM_017492.2 | CARS | NM_001751.3 | ATP6V1E1 | NM_001696.2 |
| CSF2RB | NM_000395.1 | GCLC | NM_001498.2 | KIAA0746 | NM_015187.1 |
| LOC400558 | XM_378631.1 | VEGFC | NM_005429.2 | MCM8 | NM_032485.4 |
| PSAT1 | NM_021154.3 | CCL2 | NM_002982.2 | USP4 | NM_003363.2 |
| MBNL2 | NM_144778.1 | F2RL3 | NM_003950.1 | SBP1 | NM_178121.1 |
| RAMP1 | NM_005855.1 | MGC15875 | NM_032921.1 | HOXA6 | NM_024014.2 |
| PLA2G10 | NM_003561.1 | PDE4C | NM_000923.1 | MTND3 | NM_173710.1 |
| LOC399788 | XM_374817.1 | HDLBP | NM_005336.2 | PPP2R3A | NM_002718.3 |
| IGSF8 | NM_052868.1 | MLLT1 | NM_005934.2 | PHLDA3 | NM_012396.1 |
| MGC57858 | NM_178508.2 | MGC20446 | NM_153611.2 | CCNT2 | NM_058241.1 |
| MGC33894 | NM_152914.1 | NOX1 | NM_007052.3 | PLXDC2 | NM_032812.7 |
| VN1R2 | NM_173856.1 | C9orf25 | NM_147202.1 | C14orf127 | NM_025152.1 |
| SEC22L1 | NM_004892.3 | OLIG3 | NM_175747.2 | LOC401488 | XM_379617.1 |
| LXN | NM_020169.2 | ADCY3 | NM_004036.2 | DISP2 | NM_033510.1 |
| ELL | NM_006532.1 | LOXL2 | NM_002318.1 | SEMA3F | NM_004186.2 |
| STAT6 | NM_003153.3 | FKBP5 | NM_004117.2 | CTBS | NM_004388.1 |
| C14orf31 | NM_152330.2 | B3GAT1 | NM_054025.1 | MYH9 | NM_002473.2 |
| NTAN1 | NM_173474.2 | JWA | NM_006407.2 | LOC93109 | NM_138399.2 |
| GLRX2 | NM_016066.3 | ATOH8 | NM_032827.3 | PSFL | NM_031301.1 |
| FN1 | NM_002026.1 | KIAA0605 | NM_014694.2 | ODF2 | NM_153437.1 |
| CAST | NM_173060.1 | CSAD | NM_015989.3 | SCARA3 | NM_016240.2 |
| LOC169355 | NM_194294.1 | LAMA3 | NM_198129.1 | TGFB3 | NM_003239.1 |
| LOC196463 | NM_173542.2 | LOC401026 | XM_376160.1 | FTHFSDC1 | NM_015440.3 |
| HEXB | NM_000521.2 | DSCR1L2 | NM_013441.2 | TINF2 | NM_012461.1 |
| FLJ12529 | NM_024811.2 | NOXA1 | XM_351868.1 | SETBP1 | NM_015559.1 |
| ZNF524 | NM_153219.2 | NOD9 | NM_024618.2 | HLX1 | NM_021958.2 |
| CHIC2 | NM_012110.2 | TNIP3 | NM_024873.2 | MGC22014 | XM_351441.1 |
| MRC2 | NM_006039.1 | LOC388504 | XM_373793.1 | SLC22A12 | NM_153378.1 |
| | | | | | |

| Gene code | Accesion No | Gene code | Accesion No | Gene code | Accesion No |
|---|---|---|---|---|---|
| PLP2 | NM_002668.1 | TMSL3 | NM_183049.1 | IMPDH1 | NM_183243.1 |
| ANAPC2 | NM_013366.3 | NPIP | NM_006985.1 | BACE | NM_138973.1 |
| HAX1 | NM_006118.2 | LOC285043 | XM_379085.1 | LGALS1 | NM_002305.2 |
| KLHL8 | NM_020803.3 | LOC388940 | XM_373980.1 | PADI1 | NM_013358.1 |
| DIA1 | NM_000398.3 | BAIAP2 | NM_006340.1 | LOC388690 | XM_373865.2 |
| UBXD4 | NM_181713.3 | STCH | NM_006948.3 | RRAGC | NM_022157.2 |
| ANG | NM_001145.2 | HRI | NM_014413.2 | KIAA0924 | XM_375471.1 |
| TPM2 | NM_003289.2 | KCTD13 | NM_178863.2 | TRAM1 | NM_014294.3 |
| DPYSL5 | NM_020134.2 | KCNJ2 | NM_000891.2 | LOC338755 | XM_291980.3 |
| ZBTB4 | NM_020899.2 | FLJ20006 | NM_017618.1 | LOC389050 | XM_374014.1 |
| MGC13024 | NM_152288.1 | LOC388278 | XM_373687.1 | LOC145853 | XM_096885.6 |
| LOC387749 | XM_370606.1 | DBN1 | NM_080881.1 | FLJ90798 | NM_153367.1 |
| HDAC8 | NM_018486.1 | FLJ36766 | NM_182623.1 | FLJ14464 | NM_032789.1 |
| CRSP3 | NM_004830.2 | NPEPL1 | NM_024663.2 | GAA | NM_000152.2 |
| LOC389739 | XM_372100.1 | TXNL4 | NM_006701.2 | LOC90410 | NM_174887.2 |
| SPRY1 | NM_199327.1 | ARHGEF17 | NM_014786.2 | LIMS2 | NM_017980.2 |
| SESN2 | NM_031459.3 | LASS5 | NM_147190.1 | TAF10 | NM_006284.2 |
| LOC401886 | XM_377480.1 | SPRED2 | NM_181784.1 | MFAP2 | NM_002403.2 |
| CLN2 | NM_000391.2 | PRICKLE2 | XM_093799.2 | ADCY6 | NM_020983.2 |
| MT1H | NM_005951.1 | PHF1 | NM_002636.3 | STOML1 | NM_004809.3 |
| FLJ13868 | NM_022744.1 | NPD007 | NM_020684.2 | C21orf97 | NM_021941.1 |
| EIIs1 | NM_152793.1 | MAGEL2 | NM_019066.2 | FLJ14011 | NM_022103.2 |
| USP47 | NM_017944.2 | CTDSP2 | NM_005730.2 | MSCP | NM_016612.1 |
| STK10 | NM_005990.1 | TGFBI | NM_000358.1 | LOC283710 | XM_211174.1 |
| LOC51145 | NM_016158.1 | SSBP2 | NM_012446.2 | CROT | NM_021151.2 |
| KIAA1036 | NM_014909.2 | NID67 | NM_032947.3 | KIAA1068 | NM_015332.2 |
| LOC400771 | XM_378890.1 | DKFZP434N1923 | NM_030974.2 | COL3A1 | NM_000090.2 |
| ATP13A | NM_020410.1 | CHPF | NM_024536.4 | COMMD3 | NM_012071.1 |
| NYREN18 | NM_016118.3 | LOC253827 | NM_198080.1 | C6orf145 | NM_183373.2 |
| PARVA | NM_018222.2 | VKORC1 | NM_024006.3 | ACTN4 | NM_004924.3 |
| | | | | | |

| Gene code | Accesion No | Gene code | Accesion No | Gene code | Accesion No |
|---|---|---|---|---|---|
| C14orf75 | NM_153046.1 | FLJ20793 | NM_019022.3 | CLN5 | NM_006493.1 |
| DHRS1 | NM_138452.1 | PCNX | NM_014982.1 | KIAA0841 | XM_049237.5 |
| SCAMP2 | NM_005697.3 | SLC28A1 | NM_004213.3 | ANKFY1 | NM_016376.2 |
| N4BP2 | NM_018177.2 | FAM8A1 | NM_016255.1 | RARA | NM_000964.1 |
| LOC91526 | NM_153697.1 | STUB1 | NM_005861.1 | NFE2L1 | NM_003204.1 |
| DKFZP434B044 | NM_031476.1 | FLJ40852 | NM_173677.1 | C6orf48 | NM_016947.1 |
| LOC115294 | NM_052937.1 | TK2 | NM_004614.2 | ACK1 | NM_005781.2 |
| APBA3 | NM_004886.2 | PRKAG1 | NM_002733.2 | KIAA1164 | NM_019092.1 |
| DNASE2 | NM_001375.1 | FLJ13815 | XM_086186.3 | ZNF440 | NM_152357.1 |
| MGC47816 | NM_173642.1 | TXNRD1 | NM_182743.1 | SCIN | NM_033128.1 |
| TAPBP | NM_003190.3 | AMFR | NM_138958.1 | ARPC2 | NM_005731.2 |
| IL13RA1 | NM_001560.2 | WDR1 | NM_005112.3 | EPIM | NM_001980.2 |
| LOC400608 | XM_375475.1 | TENS1 | NM_022748.6 | SYNE1 | NM_133650.1 |
| UGCGL2 | NM_020121.2 | MDS006 | NM_020233.3 | PIP5K2B | NM_138687.1 |
| C10orf42 | NM_138357.1 | EEF1G | NM_001404.3 | ANXA5 | NM_001154.2 |
| RP4-622L5 | NM_019118.2 | MGC9913 | XM_378178.1 | DNCL2A | NM_177953.1 |
| LOC389119 | NM_203370.1 | ITGB1 | NM_033668.1 | KIRREL | NM_018240.3 |
| RPH3AL | NM_006987.2 | MGC45386 | NM_198527.1 | TNFRSF10B | NM_147187.1 |
| ARK5 | NM_014840.1 | C16orf35 | NM_012075.1 | RPS4X | NM_001007.3 |
| MI-ER1 | NM_020948.1 | PPP2R5A | NM_006243.2 | TCEA2 | NM_003195.4 |
| LOC388952 | XM_373986.1 | D4ST1 | NM_130468.2 | DKFZp762C186 | XM_170658.1 |
| NFE2L2 | NM_006164.2 | CUL4B | NM_003588.2 | MAP4K4 | NM_004834.2 |
| KCNH4 | NM_012285.1 | CGI-72 | NM_032205.2 | HAK | NM_052947.1 |
| DKFZp313G1735 | NM_198150.1 | PRO0456 | NM_014127.1 | CXCL12 | NM_199168.1 |
| ISGF3G | NM_006084.3 | MYST1 | NM_032188.1 | C19orf10 | NM_019107.1 |
| FLJ20477 | NM_017837.2 | PLK2 | NM_006622.1 | SMYD3 | NM_022743.1 |
| SGCE | NM_003919.1 | LOC377678 | XM_352744.1 | COL4A1 | NM_001845.3 |
| FLJ11196 | NM_197958.1 | MAN1B1 | NM_007230.1 | C10orf9 | NM_145012.3 |
| C20orf108 | NM_080821.1 | FLJ90231 | NM_173581.1 | TEX261 | NM_144582.2 |
| BHLHB2 | NM_003670.1 | CLSTN1 | NM_014944.2 | GBF1 | NM_004193.1 |
| | | | | | |

| Gene code | Accesion No | Gene code | Accesion No | Gene code | Accesion No |
|---|---|---|---|---|---|
| C21orf69 | NM_058189.1 | TRIM16 | NM_006470.2 | FLJ14153 | NM_022736.1 |
| SLC16A3 | NM_004207.1 | MAP1LC3B | NM_022818.2 | DUX3 | NM_012148.1 |
| DCX | NM_178153.1 | ZNF155 | NM_198089.1 | MAPK4 | NM_002747.2 |
| RAB22A | NM_020673.2 | F8A | NM_012151.2 | LOC286016 | NM_203419.1 |
| RAB5B | NM_002868.2 | HSPC072 | NM_014162.1 | STX12 | NM_177424.1 |
| MGAT4B | NM_014275.2 | LOC374596 | XM_350967.1 | AP1M1 | NM_032493.2 |
| RHBDF1 | NM_022450.2 | MGC29956 | NM_144638.1 | CHRAC1 | NM_017444.3 |
| C9orf80 | NM_021218.1 | FLJ39035 | NM_182580.1 | EMS1 | NM_138565.1 |
| PIM1 | NM_002648.2 | PRKCI | NM_002740.3 | LOC57146 | NM_020422.3 |
| MTVR1 | NM_152832.1 | PLA2G6 | NM_003560.1 | WDR23 | NM_181357.1 |
| HEBP1 | NM_015987.2 | ECGF1 | NM_001953.2 | DOC-1R | NM_005851.3 |
| AIRE | NM_000383.1 | LOC91978 | XM_042012.4 | AES | NM_001130.5 |
| PDLIM1 | NM_020992.2 | CHKL | NM_005198.3 | PL6 | NM_007024.4 |
| RPL13 | NM_000977.2 | ATF4 | NM_182810.1 | C9orf89 | NM_032310.2 |
| NCOR1 | NM_006311.2 | KIAA1442 | XM_044921.4 | DCP1B | NM_152640.3 |
| DNAJC13 | NM_015268.1 | IGBP1 | NM_001551.1 | CTSB | NM_001908.2 |
| TSC2 | NM_021056.1 | NSD1 | NM_022455.3 | TLE2 | NM_003260.3 |
| RNF149 | NM_173647.2 | AIP | NM_003977.1 | RPL9 | NM_000661.2 |
| LOC388536 | XM_371163.2 | ORC5L | NM_002553.2 | DKK3 | NM_013253.3 |
| PHCA | NM_018367.3 | KLF16 | NM_031918.1 | SAS | NM_005981.3 |
| TEX27 | NM_021943.1 | AMPD2 | NM_139156.1 | OBRGRP | NM_017526.2 |
| RRP4 | NM_014285.4 | SLC2A4RG | NM_020062.3 | UPLC1 | NM_017707.2 |
| ZFP64 | NM_199427.1 | KIAA0882 | XM_093895.6 | MRPL24 | NM_024540.2 |
| APG16L | NM_030803.5 | TCEA1 | NM_006756.2 | GNB2 | NM_005273.2 |
| ENG | NM_000118.1 | LOC399972 | XM_378321.1 | RXRB | NM_021976.3 |
| CD81 | NM_004356.2 | DHRS8 | NM_016245.1 | VMP1 | NM_030938.2 |
| DOK1 | NM_001381.2 | G1P2 | NM_005101.1 | CASP10 | NM_032974.1 |
| FKBP1A | NM_000801.2 | TPST1 | NM_003596.2 | KIAA0913 | NM_015037.1 |
| LOC64744 | NM_022733.1 | UQCRB | NM_006294.2 | CAMLG | NM_001745.2 |
| ARAF1 | NM_001654.1 | LOC88523 | NM_033111.2 | PNAS-4 | NM_016076.2 |
| | | | | | |

| Gene code | Accesion No | Gene code | Accesion No | Gene code | Accesion No |
|---|---|---|---|---|---|
| OCIA | NM_017830.1 | DPAGT1 | NM_001382.2 | APBA1 | NM_001163.2 |
| LOC400555 | MM_375379.1 | FLJ12078 | NM_024977.1 | NFATC3 | NM_173164.1 |
| PH-4 | NM_177938.1 | CXXC1 | NM_014593.1 | PRO1843 | NM_018507.1 |
| FKBP11 | NM_016594.1 | FLJ21127 | NM_024549.3 | DDIT4 | NM_019058.1 |
| DDEF2 | NM_003887.1 | FLNA | NM_001456.1 | DNCH1 | NM_001376.2 |
| SH3GLB1 | NM_016009.2 | SDFR1 | NM_017455.1 | SLC22A18 | NM_002555.3 |
| CREB3 | NM_006368.4 | COL9A2 | NM_001852.3 | HSPA12B | NM_052970.3 |
| C1orf8 | NM_004872.3 | RPL29 | NM_000992.2 | C20orf177 | NM_022106.1 |
| MGC42090 | NM_152774.1 | HARS | NM_002109.3 | GLTSCR2 | NM_015710.2 |
| D2S448 | XM_056455.3 | RPL32 | NM_000994.2 | BARHL2 | NM_020063.1 |
| RPL13A | NM_012423.2 | LOC388291 | XM_373692.1 | RPL41 | NM_021104.1 |
| LOC127262 | NM_182752.2 | FBXO18 | NM_178150.1 | KIAA0186 | NM_021067.1 |
| EEF1 D | NM_001960.2 | PTK9L | NM_007284.3 | RELB | NM_006509.2 |
| NMT2 | NM_004808.1 | HNOEL-iso | NM_020190.1 | SERPINH1 | NM_001235.2 |
| KIAA0472 | XM_290898.2 | MGC21874 | XM_291105.1 | ABC1 | NM_022070.3 |
| ICA1 | NM_022308.1 | KDELR3 | NM_006855.2 | GRN | NM_002087.1 |
| DEGS | NM_144780.1 | MDS032 | NM_018467.1 | USP52 | NM_014871.2 |
| TRAPPC3 | NM_014408.3 | FLJ10486 | NM_018109.2 | FLJ14281 | NM_024920.3 |
| NFKBIL2 | NM_013432.3 | LOC90353 | NM_145232.2 | SEMA3C | NM_006379.2 |
| C17orf37 | NM_032339.3 | E2F6 | NM_198257.1 | PLCD1 | NM_006225.1 |
| PLEKHA1 | NM_021622.2 | ARL6IP4 | NM_018694.1 | ZNF578 | NM_152472.1 |
| COX15 | NM_004376.3 | LYK5 | NM_153335.3 | LOC221143 | NM_174928.1 |
| RPS14 | NM_005617.2 | PXN | NM_002859.1 | LTBP1 | NM_000627.1 |
| LEPREL2 | NM_014262.2 | KIAA0992 | NM_016081.2 | FLJ32965 | NM_182506.1 |
| ARHGEF11 | NM_014784.2 | KIAA0628 | NM_014789.1 | MGC33338 | NM_152366.2 |
| TREX2 | NM_017518.3 | SCDR10 | NM_198704.1 | CORO1C | NM_014325.2 |
| PPP2R2B | NM_181677.1 | LOC169611 | NM_182487.1 | LOC401483 | XM_376800.1 |
| MGC22805 | NM_144590.1 | C11orf1 | NM_022761.1 | RAPGEFL1 | NM_016339.1 |
| RNPEP | NM_020216.3 | LOC199800 | XM_373810.2 | MOSPD3 | NM_023948.3 |
| MGC21518 | NM_145274.1 | ZNF499 | NM_032792.2 | FVT1 | NM_002035.1 |
| | | | | | |

| Gene code | Accesion No | Gene code | Accesion No | Gene code | Accesion No |
|---|---|---|---|---|---|
| PLXNB2 | XM_371474.1 | | | | |
| TP53 | NM_000546.2 | | | | |
| HIP14 | NM_015336.1 | | | | |
| FLJ11078 | NM_018316.1 | | | | |
| M6PRBP1 | NM_005817.2 | | | | |
| LOC90624 | NM_181705.1 | | | | |
| RALA | NM_005402.2 | | | | |
| ASPSCR1 | NM_024083.2 | | | | |
| PCDHB9 | NM_019119.3 | | | | |
| LOC161247 | NM_203402.1 | | | | |
| FLJ20531 | NM_017865.2 | | | | |
| PTK7 | NM_152881.2 | | | | |
| SARDH | NM_007101.2 | | | | |
| MGC4238 | NM_032332.2 | | | | |
| PIGS | NM_033198.2 | | | | |
| FLJ10803 | NM_018224.1 | | | | |
| OAZ1 | NM_004152.2 | | | | |
| SPUF | NM_013349.3 | | | | |
| TNFSF4 | NM_003326.2 | | | | |
| BANF1 | NM_003860.2 | | | | |
| FLJ22471 | NM_025140.1 | | | | |

### Example 9: Formation of capillary tubes

Angiogenesis is the generation and formation of new capillary blood vessels, a process fundamental for processes like wound healing and reproduction. It's also involved in pathological processes (rheumatoid arthritis, tumor growth and metastasis).

### Principle

In this case the assay is set up in order to test the influence of diverse compounds (i.e. chemical compounds or miRNA etc.) on tube formation by CMPC (cardio mycocyte progenitor cells). In order for these cells to form tubes these cells have to differentiate into smooth muscle cells and endothelial cells.

The formation of these newly formed capillary tubes is visualised by staining α-SMA (α smooth muscle actin) on smooth muscle cells and PECAM-1 (platelet endothelial cell adhesion molecule-1, CD31) on endothelial cells.

### Materials and Methods

### Reagents

a. CMPC (adult or fetal cardio mycocyte progenitor cells)
b. EBM-2 (endothelial cell basal medium). Clonetics, Cat. No. CC-3156
c. EGM-2: EBM-2 + EGM-2 single qouts Cat. No. CC-4176 + 2% FBS
d. VEGF (vascular endothelial growth factor) S
e. 24-well cell culture cluster, flat bottom with lid. Corning Costar Cat. No. 3524
f. Microscope cover glasses 12mm Ø No.1 Cat. No. 01 115 20 Marienfeld GmbH & Co.KG.
g. Monoclonal anti actin, α-Smooth muscle, antibody produced in mouse. Sigma Cat. No. A2547 .2ml clone 1A4.
h. Alexa Fluor 488, goat anti mouse SFX kit. Invitrogen Cat. No. A31619
i. PECAM-1 (C-20) goat polyclonal IgG. Santa Cruz Biotechnology Cat. No. sc-1505 lot no. B169.
j. Donkey anti goat Cy3
k. Hoechst
l. Buffer Hoechst
m. In vitro angiogenesis assay kit. Chemicon International Cat. No. ECM625
n. PBS (phosphate buffered saline)
o. Water (demineralised)
p. Block buffer: 0.1% Saponin (supplier, Cat. No.) and 2% BSA (Sigma, Cat. No.) in PBS
q. 4% Para formaldehyde
r. Moviol

### Apparatus

a. pipettes
b. microscopy
c. tweezers
d. Incubator 37° Celsius 5%CO₂
e. Humidified box
f. Tissues
g. 200 µl pipette tips
h. scissors
i. refirdgerator
j. freezer
k. parafilm

### In Vitro Angiogenesis assay

a. Make use of CMPC (adult/fetal)
b. Cells are cultured in SP++ (SP++: see reagent (c) in the section above, i.e. EBM-2 + EGM-2 single qouts Cat. No. CC-4176 + 2% FBS). For cultivation use a split ratio between 1:3 and 1:10. Cells can be grown until passage number 22-24 after passage 22-24 the phenotype of the cell will change.

### Day before start of experiment:

a. Place sterilised (either by temperature or EtOH) 12mm Ø microscope cover glasses into 24 well plate(s)
b. Put 24-well plate(s) together with 200µl pipette tips at -20°C overnight.
c. Thaw ECMatrix gel solution overnight on ice at 4°C

### Start of experiment

a. Sterilise scissors in flow cabinet.
b. Place 24-well plate on ice in flow cabinet
c. Place ice cold 200µl pipette tips in flow cabinet
d. Keep ECMatrix on ice before usage.
e. Cut off tip of 200 µl pipette tip and pipette 90µl ECMatrix into one well of a 24-well plate. (work as quick and cold as possible) Repeat until all wanted wells are coated.
f. Make sure ECMatrix is even distributed in each well, then put plate for minimal one hour at 37°C

### Intermezzo

If test material is miRNA or DNA transfect cells before seeding cells onto ECMatrix If test material is a chemical compound just ad compound after/during seeding cells. Always take proper controls
g. After one hour seed 15.000 cell/well (0.5ml/well) in a 24-well ECMatrix coated plate in EGM-2 medium with * %FBS with an extra addition of 100 ng/ml VEGF. Incubate cells for 24h at 37°C and 5% CO₂. 0.5% - 10%
h. If vessel formation has occurred take pictures before going further. This in order to safe and ensure results if anything goes wrong during staining.

### Staining for α-SMA and PECAM-1

a. Remove supernatant and fixate cells with 4% Para formaldehyde during 15 minutes at RT 500µl/well.
b. Wash twice with 1 ml PBS
c. Block 30 minutes with blocking buffer 500µl/well
d. Meanwhile prepare primary anti body solution. Both primary antibodies can be used at the same time on the same sample.
e. Use 50 µl primary antibody solution for one sample (is one Microscope cover glasses 12mm Ø). Dilute PECAM-1 1:200 in block buffer and α-SMA 1:40 in block buffer.
f. Pipette 50µl primary antibody solution onto parafilm.
g. Take the Microscope cover glass(es) out of the 24-well plate with a pair of tweezers. Put upside down on the 50µl antibody solution(s).
h. Place parafilm with samples in humidified box and incubate overnight at 4°C.
i. After incubation place microscope cover glasses back into a 24 well plate and wash 3 times with 1 ml PBS.
j. During last wash step prepare the first secondary antibody solution by diluting 1:400 Cy3 donkey anti-goat in PBS. Again 50µl for each sample is used.
   *While working with secondary antibodies keep samples protected from light as much as possible.*
k. Pipette 50µl first secondary antibody solution onto parafilm and add microscope cover glasses. (see step g)
l. Incubate during 1 hour at RT in the dark.
m. After incubation place Microscope cover glasses back into 24-well plate and wash 3 times with 1ml PBS
n. During last wash step prepare the second secondary antibody solution by diluting 1:400 488nm goat anti-mouse in PBS. Again 50µl for each sample is used.
o. Pipette 50µl second secondary antibody solution onto parafilm and add microscope cover glasses. (see step g)
p. Incubate at room temperature for 1 hour.

The results of an in vitro angiogenesis assay are depicted in Figure 8.

### Example 10: Human Foetal Cardiomyocyte Progenitor cells Improve Left Ventricular Systolic Function after Myocardial Infarction in NOD/scid Mice

Recently, the existence of cardiomyocyte progenitor cells (CMPCs) that reside in the heart became appreciated. We isolated CMPCs from human foetal heart and showed that these human CMPCs (hCMPCs) can differentiate into functional cardiomyocytes in vitro. In the present study we investigated whether these cells are able to engraft in the ischemic myocardium and improve left ventricular function in an immune-compromised mouse myocardial infarction model.

*Methods:* hCMPCs were isolated from human fetal hearts by magnetic cell sorting based on expression of SCA-1. Myocardial infarction (MI) was induced in immune-compromised NOD/scid mice. Twenty minutes after MI, hCMPCs labelled with eGFP (hCMPC group, 2.0 x 10^5 cells in 20µl, n=11) or vehicle only (MI+Medium group, n=12) were injected into the infarcted area. Sham operated mice (Sham) were used as baseline (n=10). Two and 14 days after induction of MI, cardiac function was serially assessed using a vertical 9.4T animal MRI. Mice were then sacrificed and the engraftment and differentiation of injected cells was assessed by immunohistochemistry.

*Results:* At day 2,, LV volumes were higher and EF (ejection fraction) was lower in both MI groups as compared to Sham, with no difference between the MI groups (Fig. 9). However at day 14, EF (ejection fraction) was higher and ESV (end systolic volume) was lower in the hCMPC group as compared to the MI+Medium group (P=0.001 and P=0.048 respectively). Although there was a trend towards a reduced EDV (end diastolic volume), this did not reach significance (P=0.14).

*Conclusions:* Foetal hCMPC engraft in the acutely infarcted myocardium, and improve LV systolic function. These results indicate the potential of hCMPC to be used in cell-based therapy for the treatment of IHD.

### Example 11 : Formation of adipocytes

CMPCs are seeded and cultured until they have reached a confluence of 90 - 100% in SP++ (SP++: EBM-2 + EGM-2 single qouts Cat. No. CC- 4176 + 2% FBS). Subsequently, the medium is replaced by DMEM 4.5g/l glucose + Na pyruvat containing 10% FBS, 1 uM dexamethasone, 0.5mM 3-Isobutyl-1- methylxanthine (IBMX), 10 ug/ml Insulin, 0.2 mM indomethacin and Pen/strep. After some days, droplets of fat are deposited around the nucleus. Every 3 days the medium is refreshed.

To confirm adipocyte differentiation adipocytes were stained with Oil red according to the following protocol:
0.7 g Oil Red O stock solution FW 408.5, Sigma O-0625 was mixed with 200 ml Isopropanol; stirred O/N, then filtered with 0.2 im and stored at +4°C. The Oil Red O

Working Solution was prepared from 6 parts Oil Red O stock; 4 parts dH2O; mixed and let sit at room temperature for 20 min and filtered with 0.2 im.

Most of the medium was removed and cells were fixated by adding 4% paraformaldehyde. The mixture was incubated for 5 min at room temperature, the formalin discarded and the same volume of fresh formalin was added. After incubation for at least 1 hour the cells can be kept in formalin for a couple of days before staining. Parafilm was wrapped around the plate to prevent drying and covered with aluminum foil. All the formalin was removed with a small transfer pipette and the wells are washed with 60% isopropanol. To the completely dry wells Oil Red O working solution was added for 10 min without touching the walls of the wells All Oil Red O was removed and immediately dH2O was added followed by washing with H2O 4 times. Then pictures were taken.

To further confirm adipocyte differentiation in vitro, RNA was isolated from differentiated and undifferentiated CMPCs. RT-PCR was performed on Leptin ((from the Greek leptos, meaning thin) is a protein hormone with important effects in regulating body weight, metabolism and reproductive function. Leptin is expressed predominantly by adipocytes, which fits with the idea that body weight is sensed as the total mass of fat in the body), Adipsin (is serine protease that is secreted by adipocytes), PPAR γ 2 (is predominantly expressed in fat tissue), GLUT 4 (glucose carriers in muscle and adipose tissues) & CYR61/CCN1 (expression decreased markedly during osteogenic differentiation, adipogenic differentiation and chondrogenic differentiation).

### Which resulted in

| | HFH | hMSC |
|---|---|---|
| leptin | N/A | up |
| adipsin | up | up |
| pparγ2 | up | up |
| Cnn1/cyr61 | down | down |
| glut4 | --- | --- |

Therefore it can be concluded that CMPCs act in the same way as hMSC when differentiated in vitro. Therefore, the CMPCs of the invention are capable of differentiating into adipocytes. The results are shown in Figure 10a to 10f.

### Cited references

1. Emanueli,C., Lako,M., Stojkovic,M. & Madeddu,P. In search of the best candidate for regeneration of ischemic tissues: are embryonic/fetal stem cells more advantageous than adult counterparts? Thromb. Haemost. 94, 738-749 (2005).
2. Leri,A., Kajstura,J. & Anversa,P. Cardiac stem cells and mechanisms of myocardial regeneration. Physiol Rev. 85, 1373-1416 (2005).
3. Smits,A.M., van Vliet,P., Hassink,R.J., Goumans,M.J. & Doevendans,P.A. The role of stem cells in cardiac regeneration. J. Cell Mol. Med. 9, 25-36 (2005).
4. Van Laake,L.W., Van Hoof,D. & Mummery,C.L. Cardiomyocytes derived from stem cells. Ann. Med. 37, 499-512 (2005).
5. Fukuda,K. & Fujita,J. Mesenchymal, but not hematopoietic, stem cells can be mobilized and differentiate into cardiomyocytes after myocardial infarction in mice. Kidney Int. 68, 1940-1943 (2005).
6. Mangi,A.A. et al. Mesenchymal stem cells modified with Akt prevent remodeling and restore performance of infarcted hearts. Nat. Med. 9, 1195-1201 (2003).
7. Orlic,D. et al. Bone marrow cells regenerate infarcted myocardium. Nature 410, 701-705 (2001).
8. Beltrami,A.P. et al. Adult cardiac stem cells are multipotent and support myocardial regeneration. Cell 114, 763-776 (2003).
9. Behfar,A. et al. Stem cell differentiation requires a paracrine pathway in the heart. FASEB J. 16, 1558-1566 (2002).
10. Menard,C. et al. Transplantation of cardiac-committed mouse embryonic stem cells to infarcted sheep myocardium: a preclinical study. Lancet 366, 1005-1012 (2005).
11. Rubart,M. et al. Physiological coupling of donor and host cardiomyocytes after cellular transplantation. Circ. Res. 92, 1217-1224 (2003).
12. Muller-Ehmsen, J. et al. Rebuilding a damaged heart: long-term survival of transplanted neonatal rat cardiomyocytes after myocardial infarction and effect on cardiac function. Circulation 105, 1720-1726 (2002).
13. Schachinger,V. et al. Transplantation of progenitor cells and regeneration enhancement in acute myocardial infarction: final one-year results of the TOPCARE-AMI Trial. J. Am. Coll. Cardiol. 44, 1690-1699 (2004).
14. Menasche,P. et al. Autologous skeletal myoblast transplantation for severe postinfarction left ventricular dysfunction. J. Am. Coll. Cardiol. 41, 1078-1083 (2003).
15. Smits,P.C. et al. Catheter-based intramyocardial injection of autologous skeletal myoblasts as a primary treatment of ischemic heart failure: clinical experience with six-month follow-up. J. Am. Coll. Cardiol. 42, 2063-2069 (2003).
16. Meyer,G.P. et al. Intracoronary bone marrow cell transfer after myocardial infarction: eighteen months' follow-up data from the randomized, controlled BOOST (BOne marrOw transfer to enhance ST-elevation infarct regeneration) trial. Circulation 113, 1287-1294 (2006).
17. Balsam,L.B. et al. Haematopoietic stem cells adopt mature haematopoietic fates in ischaemic myocardium. Nature 428, 668-673 (2004).
18. Murry,C.E. et al. Haematopoietic stem cells do not transdifferentiate into cardiac myocytes in myocardial infarcts. Nature 428, 664-668 (2004).
19. Mummery,C. et al. Differentiation of human embryonic stem cells to cardiomyocytes: role of coculture with visceral endoderm-like cells. Circulation 107, 2733-2740 (2003).
20. Passier,R. et al. Increased cardiomyocyte differentiation from human embryonic stem cells in serum-free cultures. Stem Cells 23, 772-780 (2005).
21. Xu,C., Police,S., Rao,N. & Carpenter,M.K. Characterization and enrichment of cardiomyocytes derived from human embryonic stem cells. Circ. Res. 91, 501-508 (2002).
22. Oh,H. et al. Cardiac progenitor cells from adult myocardium: homing, differentiation, and fusion after infarction. Proc. Natl. Acad. Sci. U. S. A 100, 12313-12318 (2003).
23. Laugwitz,K.L. et al. Postnatal isl1+ cardioblasts enter fully differentiated cardiomyocyte lineages. Nature 433, 647-653 (2005).
24. Pfister,O. et al. Circ. Res. 97, 52-61 (2005).
25. Messina,E. et al. Isolation and expansion of adult cardiac stem cells from human and murine heart. Circ. Res. 95, 911-921 (2004).
26. Slager,H.G., Van Inzen,W., Freund,E., Van den Eijnden-Van Raaij AJ & Mummery,C.L. Transforming growth factor-beta in the early mouse embryo: implications for the regulation of muscle formation and implantation. Dev. Genet. 14, 212-224 (1993).
27. Takahashi,T. et al. Ascorbic acid enhances differentiation of embryonic stem cells into cardiac myocytes. Circulation 107, 1912-1916 (2003).
28. Sachinidis,A. et al. Cardiac specific differentiation of mouse embryonic stem cells. Cardiovasc. Res. 58, 278-291 (2003).
29. Li,T.S. et al. Regeneration of infarcted myocardium by intramyocardial implantation of ex vivo transforming growth factor-beta-preprogrammed bone marrow stem cells. Circulation 111, 2438-2445 (2005).
30. ten Dijke,P. & Hill,C.S. New insights into TGF-beta-Smad signalling. Trends Biochem. Sci. 29, 265-273 (2004).
31. Inman,G.J. et al. SB-431542 is a potent and specific inhibitor of transforming growth factor-beta superfamily type I activin receptor-like kinase (ALK) receptors ALK4, ALK5, and ALK7. Mol. Pharmacol. 62, 65-74 (2002).
32. Mouquet,F. et al. Restoration of cardiac progenitor cells after myocardial infarction by self-proliferation and selective homing of bone marrow-derived stem cells. Circ. Res. 97, 1090-1092 (2005).
33. Flanders,K.C., Holder,M.G. & Winokur,T.S. Autoinduction of mRNA and protein expression for transforming growth factor-beta S in cultured cardiac cells. J. Mol. Cell Cardiol. 27, 805-812 (1995).
34. van der Heyden,M.A. et al. P19 embryonal carcinoma cells: a suitable model system for cardiac electrophysiological differentiation at the molecular and functional level. Cardiovasc. Res. 58, 410-422 (2003).
35. Goumans,M.J. et al. Balancing the activation state of the endothelium via two distinct TGF-beta type I receptors. EMBO J. 21, 1743-1753 (2002).

## Claims

1. A cell fraction comprising at least 90 % human cardiomyocyte progenitor cells (CMPCs) which are **characterized by** a Sca-1 epitope and CD31 on their cell surface.

2. An in vitro method for the enrichment of human cardiomyocyte progenitor cells (CMPCs), comprising the steps of:
(a) dissociating heart tissue, e.g. derived from the atrium; and
(b) enrichming CMPCs with a Sca-1 binding agent and a CD31 binding agent.

3. A cell fraction comprising CMPCs according to claim 1, which is obtained by a method of claim 2.

4. A cell fraction comprising CMPCs according to claim 1, said cells being capable of differentiation into cardiomyocytes in vitro after 5-azacytidine or 5-aza-2'-deoxycytidine treatment in the presence of ascorbic acid.

5. An in vitro method for the differentiation of human CMPCs into cardiomyocytes, preferably in the absence of co-cultured neonatal cardiomyocytes, comprising the steps of:
(a) providing a cell fraction comprising CMPCs according to claim 1; and
(b) treating said CMPCs with a demethylating agent.
(c) allowing the so-treated CMPCs to differentiate into cardiomyocytes.

6. The method of claim 5, wherein said demethylating agent is 5-azacytidine or 5-aza-2'-deoxycytidine.

7. The method of claim 5 or claim 6, wherein said cell fraction comprising CMPCs is treated with said demethylating agent in the presence of an antioxidation agent, preferably ascorbic acid.

8. The method of according to any one of claims 5-7, further comprising the step of:
(b1) treating the CMPCs with a TGF-β family member.

9. The method of claim 8, wherein said TGF-β family member is TGF-β.

10. A pharmaceutical composition comprising the cell fraction according to claim 1.

11. A cell fraction according to claim 1, for use in a cardiomyocyte replacement therapy and/or in a method for the treatment of myocardial infarction or for ameliorating the effects of myocardial infarction.

12. A cell fraction for use according to claim 11, wherein the cardiomyocyte replacement therapy is the improvement of cardiac contractile force.

13. Use of a cell fraction according to claim 1, for screening methods, comprising the steps of:
(a) providing said cell fraction comprising cardiomyocyte progenitor cells (CMPCs);
(b) bringing said CMPCs into contact with a test substance; and
(c) evaluating the effect of said test substance on the differentiation capability of said CMPCs.

14. Use according to claim 13, wherein said screening method is a drug screening method.

## Patentansprüche

1. Zellfraktion, umfassend mindestens 90 % menschliche Kardiomyozytenvorläuferzellen (CMPCs), die durch ein Sca-1-Epitop und CD31 auf ihrer Zelloberfläche gekennzeichnet sind.

2. In vitro-Verfahren zur Anreicherung menschlicher Kardiomyozytenvorläuferzellen (CMPCs), umfassend die Schritte von:
(a) Dissoziieren von Herzgewebe, z. B. abgeleitet von Atrium; und
(b) Anreichern von CMPCs mit einem Sca-1-Bindungsagens und einem CD31-Bindungsagens.

3. Zellfraktion, umfassend CMPCs nach Anspruch 1, erhalten durch ein Verfahren nach Anspruch 2.

4. Zellfraktion, umfassend CMPCs nach Anspruch 1, wobei die Zellen in der Lage sind, nach einer Behandlung mit 5-Azacytidin oder 5-Aza-2'-deoxycytidin bei Vorhandensein von Askorbinsäure in vitro zu Kardiomyozyten zu differenzieren.

5. In vitro-Verfahren zur Differenzierung menschlicher CMPCs zu Kardiomyozyten, bevorzugt in Abwesenheit von co-kultivierten neonatalen Kardiomyozyten, umfassend die Schritte von:
(a) Bereitstellen einer Zellfraktion, umfassend CMPCs nach Anspruch 1;
und
(b) Behandeln der CMPCs mit einem demethylierenden Agens,
(c) Ermöglichen den so behandelten CMPCs zu Kardiomyozyten zu differenzieren.

6. Verfahren nach Anspruch 5, wobei das demethylierende Agens 5-Azacytidin oder 5-Aza-2'-deoxycytidin ist.

7. Verfahren nach Anspruch 5 oder Anspruch 6, wobei die CMPCs umfassende Zellfraktion mit dem demethylierenden Agens in Anwesenheit eines Antioxidationsmittels, bevorzugt Askorbinsäure, behandelt wird.

8. Verfahren nach einem der Ansprüche 5-7, ferner umfassend den Schritt von:
(b1) Behandeln der CMPCs mit einem Mitglied der TGF-β-Familie.

9. Verfahren nach Anspruch 8, wobei das Mitglied der TGF-β-Familie TGF-β ist.

10. Pharmazeutische Zusammensetzung, umfassend die Zellfraktion nach Anspruch 1.

11. Zellfraktion nach Anspruch 1 zur Verwendung in einer Therapie zum Ersetzen von Kardiomyozyten und/oder in einem Verfahren zur Behandlung eines Herzinfarkts oder zur Verbesserung der Auswirkungen eines Herzinfarkts.

12. Zellfraktion zur Verwendung nach Anspruch 11, wobei die Therapie zum Ersetzen der Kardiomyozyten die Verbesserung der Kontraktionskraft des Herzens ist.

13. Verwendung einer Zellfraktion nach Anspruch 1 für Screening-Verfahren, umfassend die Schritte von:
(a) Bereitstellen der Zellfraktion, umfassend Kardiomyozytenvorläuferzellen (CMPCs);
(b) in Kontakt Bringen der CMPCs mit einer Testsubstanz; und
(c) Auswerten der Auswirkung der Testsubstanz auf die Differenzierungsfähigkeit der CMPCs.

14. Verwendung nach Anspruch 13, wobei das Screening-Verfahren ein Verfahren zum Screening von Arzneimitteln ist.

## Revendications

1. Fraction de cellule comprenant 90 % au moins de cellules progénitrices de cardiomyocytes humains (CMPC) qui sont **caractérisées par** un épitope de Sca - 1 et CD31 sur leur surface cellulaire.

2. Procédé *in vitro.* destiné à enrichir des cellules souches de cardiomyocytes humains (CMPCs), comprenant les étapes consistant à :
(a) dissocier un tissu cardiaque, obtenu par exemple à partir de l'oreillette ; et
(b) enrichir les CMPC avec un agent de liaison de Sca - 1 et un agent de liaison de CD31.

3. Fraction de cellule comprenant des CMPC selon la revendication 1, qui est obtenue grâce à un procédé selon la revendication 2.

4. Fraction de cellule comprenant des CMPC selon la revendication 1, lesdites cellules étant capables d'une différenciation dans des cardiomyocytes *in vitro* après un traitement par 5 - azacytidine ou par 5 - aza - 2' - désoxycytidine en présence d'acide ascorbique.

5. Procédé *in vitro* de différentiation de CMPC humaines en cardiomyocytes, de préférence en l'absence de cardiomyocytes néonatals cultivés conjointement, comprenant les étapes consistant à :
(a) fournir une fraction de cellule comprenant des CMPC selon la revendication 1 ; et
(b) traiter lesdites CMPC avec un agent de déméthylation ;
(c) permettre aux CMPC ainsi traitées de se différencier en cardiomyocytes.

6. Procédé selon la revendication 5, dans lequel ledit agent de déméthylation est 5 - azacytidine ou 5 - aza - 2' - désoxycytidine.

7. Procédé selon la revendication 5 ou la revendication 6, dans lequel la dite fraction de cellule qui comprend des CMPC est traitée avec ledit agent de déméthylation en présence d'un agent antioxydant, de préférence de l'acide ascorbique.

8. Procédé selon l'une quelconque des revendications 5 à 7, comprenant en outre une étape consistant à :
(b1) traiter les CMPC avec un élément de la famille de TGF - β.

9. Procédé selon la revendication 8, dans lequel ledit élément de la famille TGF - β est le TGF - β.

10. Composition pharmaceutique comprenant la fraction de cellule selon la revendication 1.

11. Fraction de cellule selon la revendication 1, destinée à une utilisation dans une thérapie de remplacement de cardiomyocytes et / ou dans un procédé destiné au traitement de l'infarctus du myocarde ou à une amélioration des effets de l'infarctus du myocarde.

12. Fraction de cellule destinée à une utilisation selon la revendication 11, dans laquelle la thérapie de remplacement de cardiomyocyte consiste à améliorer la force de contraction cardiaque.

13. Utilisation d'une fraction de cellule selon la revendication 1, destinée à des procédés de dépistage, comprenant les étapes consistant à :
(a) fournir ladite fraction de cellule qui comprend des cellules souches de cardiomyocytes (CMPC) ;
(b) mettre en contact lesdites CMPC avec une substance d'essai ; et
(c) évaluer l'effet de ladite substance d'essai en ce qui concerne les possibilités de différenciation en CMPC.

14. Utilisation selon la revendication 13, dans laquelle ledit procédé de dépistage est un procédé de dépistage de drogue.
